# EUROPÄISCHE PATENTANMELDUNG

(11) **EP 2 441 826 A1**
(43) Veröffentlichungstag der Anmeldung: **18.04.2012**
(21) Anmeldenummer: 11185446.9
(22) Anmeldetag: 17.10.2011
(51) Int. Cl.: C12M 1/12, C12M 3/06

(54) **Verfahren zur Produktisolierung und Substratzufuhr in einem Bioreaktor**

(30) Priorität: 15.10.2010 DE 102010048422
(71) Anmelder: Melin, Thomas, 6291 AG Vaals (NL)
(72) Erfinder: Melin, Prof. Dr.-Ing., Thomas, 6291AG Vaals (NL); Büchs, Prof. Dr.-Ing., Jochen, 52064 Aachen (DE); Carstensen, Dipl.-Ing., Frederike, 52064 Aachen (DE)
(74) Vertreter: von Kreisler Selting Werner

(57) **Zusammenfassung**

Die vorliegende Erfindung betrifft ein Verfahren zur Durchführung von Reaktionen mit biologischem Aktivmaterial in einem Bioreaktor, einen Bioreaktor und die Verwendung des Bioreaktors für biochemische Reaktionen, insbesondere Fermentationen, enzymatische Umsetzungen oder der Produktion monoklonaler Antikörper.

## Beschreibung

Die vorliegende Erfindung betrifft ein Verfahren zur Durchführung von Reaktionen mit biologischem Aktivmaterial in einem Bioreaktor, einen Bioreaktor und die Verwendung des Bioreaktors für biochemische Reaktionen, insbesondere Fermentationen, enzymatische Umsetzungen oder die Produktion monoklonaler Antikörper.

Membranreaktoren werden häufig in der Biotechnologie für die Durchführung biochemischer Reaktionen eingesetzt. Der am weitesten verbreitete Membranreaktortyp folgt dem Extraktionsprinzip. Dabei ist zwischen den Grundprinzipien "selektive Produktentfernung" und "Katalysatorrückhalt" zu unterscheiden. Das Extraktionsprinzip wird sowohl in der Flüssig- als auch in der Gasphase eingesetzt, im Falle der Pervaporation findet ein Phasenübergang von flüssig zu gasförmig statt.

Das zweite Haupteinsatzgebiet von Membranreaktoren ist das Distributionsprinzip, bei dem ein Edukt mit Hilfe einer Membran gezielt der Reaktion zugeführt wird. Dabei lassen sich zwei Funktionen unterscheiden: Durch die gleichmäßige Zufuhr eines Edukts entlang des Reaktors können Hotspots und Nebenreaktionen verhindert werden. Bei Einsatz dichter Membranen kann zudem eine Komponente aus einem Gemisch selektiv der Reaktion zugegeben werden, so dass die Membran als vorgeschaltete Trennstufe wirkt.

Der erfolgreichste und am weitesten verbreitete Membranreaktor ist die mikrobische Zelle, in der eine Membran selektive Substrataufnahme und selektive Produktabgabe ermöglicht, während sie Enzyme und Coenzyme zurückhält. Seit den 1950er Jahren wird das Konzept der Kopplung einer biologischen Reaktion mit Membrantrennverfahren auch industriell angewandt, anfangs vor allem in der Milchindustrie. Aufgrund der niedrigen Temperatur- und Druckbereiche können meist kommerzielle organische Membranen verwendet werden. Die für Membranbioreaktoren (MBR) geltenden Grundprinzipien entsprechen weitgehend denen der chemischen Reaktion und lassen sich in selektive Produktentfernung, Rückhalt von Biokatalysator und selektive Substratzugabe unterteilen.

Bei biologischen Reaktionen entsteht häufig ein komplexes Produktgemisch mit nur wenigen Wertprodukten. Durch eine in-situ-Entfernung inhibierender oder toxischer Nebenprodukte können die Lebensdauer des Biokatalysators verlängert und der Durchsatz erhöht werden. Zudem ermöglichen Membranbioreaktoren eine Erhöhung der Konzentration der Mikroorganismen, die zu einer Verbesserung der Produktivität des Reaktors beiträgt.

Enzymkatalysierte Veresterungen wie beispielsweise die Synthese von Triglyceriden aus Fettsäuren und Glycerin sind gleichgewichtslimitiert und erzeugen Wasser als Nebenprodukt. In Membranbioreaktoren wird die Triglyceridausbeute durch selektiven Abzug von Wasser erhöht. Zudem können die Enzyme auf der Membranoberfläche immobilisiert werden. Eine Anreicherung von unpermeablen Nebenprodukten wie Salzen und Essigsäure, kann jedoch zum Tod der aktiven Zellen und zu Fouling der Membran führen. Periodisches Reinigen der Membran oder Vorschalten einer MF/UF-Membranstufe kann dieses Problem verringern.

Für einige Anwendungen ist es möglich, statt der unerwünschten Nebenprodukte das erwünschte Wertprodukt über eine Membran abzuziehen, um den Gleichgewichtsumsatz zu erhöhen und weitere Trennschritte zu vermeiden. Beispielsweise kann Ethanol durch kontinuierliche Fermentation hergestellt werden. Eine Abtrennung von Ethanol durch Pervaporation erhöht die volumentrische Produktivität, verringert die Toxizität gegenüber den fermentierenden Organismen und erzeugt einen hochkonzentrierten Produktstrom, was den Energiebedarf einer anschließenden Destillation verringert.

In Membranbioreaktoren kann die Membran in einer dem Reaktor nachgeschalteten Stufe oder direkt im Reaktor untergebracht sein. Im zweiten Fall ist eine Immobilisierung des Biokatalystors auf der Membranoberfläche oder in den Poren möglich. Dadurch lassen sich besonders bei Verwendung von Hohlfasermembranen längere Kontaktzeiten zwischen Reaktanden und Enzymen erreichen als mit herkömmlichen Bioreaktoren. Aufgrund der Gefahr des Membran-Foulings speziell bei Fermentationen und Anwendungen mit Mikroorganismen, ist aber die räumliche Trennung der zwei Einheiten weiter verbreitet, da dies die erforderliche Reinigung und Sterilisierung stark vereinfacht. Mechanische in-situ-Reinigungsprozesse sind erfolgreich, wenn Fouling auf Ablagerung von Partikeln beruht. Bei Biofouling durch Adsorption von Biochemikalien sind chemische Reinigungstechniken notwendig. Je nach Größe des Biokatalysators werden unterschiedliche Membranverfahren eingesetzt: NF für Coenzyme, UF für Enzyme, MF für Mikroorganismen. Enzyme werden alternativ auch auf einem löslichen Polymer befestigt, um ihre molekulare Größe (10 000 bis 100 000 Da) zu erhöhen, so dass sie auch durch MF zurückgehalten werden, während Produkte entfernt oder Edukte zugegeben werden.

Enzymatische Reaktionen werden in der Lebensmittel- und der Pharmaindustrie häufig im Batch-Betrieb durchgeführt, dabei wird das Enzym am Ende deaktiviert. Dadurch ist die Produktivität gering, die Betriebskosten sind hoch, die katalytische Aktivität geht verloren und die Produktqualität schwankt. Immobilisierung löst einige der Probleme, verringert aber die Aktivität um 10 bis 90 %. Enzymatische Membranreaktoren (EMR) ermöglichen den vollständigen Rückhalt des Enzyms beim Abzug der Reaktionsprodukte. Die Enzyme zirkulieren entweder auf der Retentatseite oder sind auf der Membranoberfläche oder in den Poren immobilisiert.

Das größte Anwendungsgebiet ist die Protein-Hydrolyse. Bei der Hydrolyse von Molkeprotein und Casein konnte durch unabhängige Kontrolle von Enzym-Substratkonzentrationen, Verweilzeit und Permeatfluss eine Produktivität erreicht werden, die um den Faktor 12 über der eines Batch-Reaktors liegt. Weitere Einsatzgebiete von EMR sind Hydrolyse und Synthese von Polysacchariden, die Klärung von Fruchtsaft oder die Umesterung von Lipiden.

Bei vielen biochemischen Prozessen übernehmen pflanzliche oder tierische Zellen, Bakterien oder Hefe die Rolle des Biokatalysators. Die Abmessungen der Biomasse liegen über 0,1 µm, so dass eine Abtrennung mit MF- oder UF-Membranen erfolgen kann. Die größten Probleme stellen Biofouling, Stofftransportlimitierung, Aktivitätsverlust und Denaturierung dar. Bei einem Vergleich von Batchreaktor und MBR für die Produktion von Milchsäure aus Lactose ergab sich, dass im MBR eine 8mal höhere Produktivität und eine 19mal höhere Biomassekonzentration erzielt wurde.

Membranen können auch zur Zugabe meist gasförmiger oder organischer Substrate zu einem wässrigen Reaktionsgemisch eingesetzt werden, was einer Kombination aus Distributor und Mehrphasenkontaktor entspricht. Anwendungen sind die sogenannte Biooxygenierung, die Biohydrogenierung oder die Zugabe lipophiler Substrate.

Eine höhere Löslichkeit von inhibierenden Substanzen in organischen Lösungsmitteln als in Wasser kann zur kontinuierlichen Abtrennung aus der wässrigen Phase genutzt werden. In konventionellen zweiphasigen Reaktoren ist der Stofftransport zwischen den Phasen durch die Grenzfläche limitiert, andererseits erschwert eine Emulgierung die Trennung. Durch Einsatz von Membranen lässt sich eine feste Grenzfläche zur Verfügung stellen, ohne die organische und die wässrige Phase innerhalb des Bioreaktors zu vermischen. Diese Prinzip wurde beispielsweise zur Reduktion von Geraniol zu Citronellol genutzt, die beide schlecht wasserlöslich sind. Geraniol, in Hexadecan gelöst, wird im Inneren einer rohrförmigen Silikonmembran durch einen Kessel geführt, der Biokatalystor und wasserlösliches Substrat in wässriger Phase enthält. Die Transformation ist vergleichbar mit dem Direktkontaktsystem, aber es entstehen keine Probleme durch Emulgierung und Bulkphasendurchbruch, die organische Lösung bleibt unkontaminiert.

Bei der Hydrolyse von Pflanzenöl in zweiphasigen Reaktoren entstehen zwei nicht mischbare Produkte: Fettsäuren gelangen in die hydrophobe Phase, Glycerin in die hydrophile Phase. Durch Einsatz eines MBR lassen sich die Produkte kontinuierlich trennen und die Enzyme zurückhalten. Das Enzym wird auf der wässrigen Seite der Membranoberfläche immobilisiert, auf der anderen Seite strömt das Pflanzenöl.

MBR mit hydrophoben Membranen werden auch zur Gasreinigung eingesetzt: Über die Gasphase wird die Biomasse mit Sauerstoff und Kohlenstoff versorgt, die wässrige Phase, in der die Mikroorganismen wachsen, stellt die anderen Nährstoffe zur Verfügung. Auf oder in der Membran bildet sich meist ein Biomassefilm. Die Membran muss eine große Kontaktfläche für Gas und Flüssigphase zur Verfügung stellen und eine hydrophobe Oberfläche besitzen, damit die organischen Moleküle abgetrennt werden können. Vorteile gegenüber konventionellen Biofiltern, in denen Gas durch ein Bett mit immobilisierten Mikroorganismen fließt, die die gasförmigen Schadstoffe abbauen, sind die kontinuierliche Eliminierung der Abbauprodukte, die hemmend wirken können, die geringere Sensibilität gegenüber Zufuhrunterbrechungen durch modularen Aufbau sowie einfacher Scale-Up.

Mikro-(MF) und Ultrafiltration (UF) sind druckbetriebene Verfahren zur Filtration von Suspensionen und Lösungen. Sie werden eingesetzt, um Feststoffe und Makromoleküle aus Flüssigkeiten abzutrennen. Je nach Größe der abzutrennenden Spezies werden die grobporigen MF-Membranen (durchschnittliche Porengröße: etwa 0,08 µm bis etwa 10 µm) oder die feinporigeren UF-Membranen (mittlerer Porendurchmesser: etwa 0,008 µm bis etwa 0,12 µm) eingesetzt. MF- und UF-Membranen haben sich in vielen industriellen Anwendungsbereichen durchgesetzt, etwa in der Umwelttechnik, metallverarbeitenden Industrie, pharmazeutischen Industrie, Lebensmittelindustrie und Biotechnik. Die Membranen können in Modulen angeordnet diskontinuierlich im Dead-End-Modus oder kontinuierlich im Cross-Flow-Modus betrieben werden oder werden in der getauchten Betriebsweise direkt in den Reaktor integriert.

Das zentrale Problem bei MF- oder UF-Membranverfahren ist die Bildung einer Deckschicht, die sich durch Ablagerung von Partikeln und größeren Molekülen auf der Membran bildet. Dadurch erhöht sich der Strömungswiderstand und es kommt zu einem Abfall des transmembranen Flusses. Betriebsweisen, bei denen ein Teil des Permeats durch die Membran zurückgepumpt wird, bieten die Möglichkeit, zumindest kurzfristig diese Deckschicht zu lösen. Sie wurden in zahlreichen Studien untersucht und kommen sowohl in konventionellen Membranbioreaktoren als auch bei der Filtration sehr verdünnter Suspensionen mit Wechsel der Filtrationsrichtung im Abstand von Sekunden - oder Millisekunden - zum Einsatz.

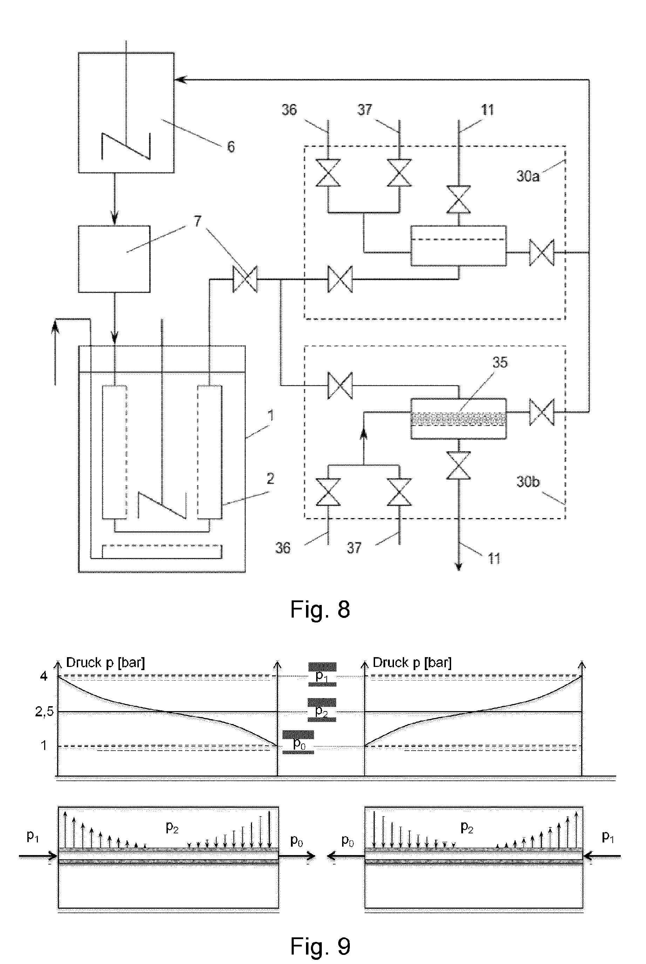

Sämtliche bisher genannte Ansätze zur Filtration, auch die mit Rückspülung, haben das Ziel, aus dem Reaktorinhalt einen möglichst großen Filtratstrom zu entnehmen, aus dem dann das Produkt isoliert werden kann oder eine Suspension aufzukonzentrieren. In keinem der genannten Fälle wird bewusst auf die Erzeugung eines positiven Netto-Filtratflusses verzichtet und demzufolge wird das Fouling-Problem durch die partielle Rückspülung zwar verringert, aber nicht gelöst. Ein Beispiel hierfür ist das in der DE 10338523 A1 offenbarte Verfahren, wo mit einer oszillierenden Pumpe auf der Suspensionsseite versucht wird das Fouling zu beherrschen, aber durch die Förderung des Feeds "gegen ein dem Fluss entgegenstehendes Hindernis", nämlich ein Druckhalteventil, ein Netto-Filtratfluss von der Suspensions- auf die Permeatseite beabsichtigt und bewirkt wird. Diese Anordnung hat als weiteren Nachteil, dass sie nur in einem extern angeordneten, zumindest über einem geschlossenen und damit durch Scherung und Sauerstoffzehrung gekennzeichneten Modul durchgeführt werden kann. Dies wird eindeutig durch die auf der Suspensionsseite beabsichtigte Druckpulsation bestätigt. Lediglich in der WO95/21687 A1 wird versucht, auf jeglichen Netto-Filtratfluss zu verzichten. Dies beruht auf einem als "Starling's Flow" bezeichneten Phänomen, das anhand von Figur 9 erläutert wird.

Voraussetzung für das Auftreten von Starling's Flow sind meist parallel geschaltete Kapillare oder Rohrmembranen, die zwei Typen von Räumen voneinander trennen: Innenräume, die mit großer Geschwindigkeit durchströmt werden, so dass in ihnen entlang der Membran ein hoher Druckabfall p1-p0 von meist über 1 bar auftritt und einen Außenraum, der druckfest ausgeführt wird und während des Betriebs keine offene Verbindung nach außen besitzt. Gemäß WO95/21687 A1 wird der Außenraum als Zylinder beschrieben, der die das Produkt erzeugenden Zellen enthält und eine große Zahl von Hohlfasermembranen umschließt. Die in diesem Raum auftretenden Strömungsgeschwindigkeiten sind gering, so dass in ihm überall ein nahezu konstanter Druck p2 herrscht, der näher am Druck p1 am Eintritt als am Druck p0 am Austritt des Innenraums und damit weit über dem Atmosphärendruck liegt. Aufgrund dieser Druckverhältnisse tritt im Eintrittsbereich des Innenraums ein Strom über die Membranen in den Außenraum und im Austrittsbereich ein Strom in umgekehrter Richtung auf. Da der Außenraum einen geschlossenen, flüssigkeitsgefüllten Raum darstellt, ist stets sichergestellt, dass kein Netto-Filtratfluss auftritt, die vom Innen- in den Außentraum tretenden Ströme, deren Größe mit den senkrechten Pfeilen in Figur 9 symbolisiert wird, also ebenso groß sind wie die in umgekehrter Richtung. Um zu vermeiden, dass dennoch lokal, also im Bereich der Strömung vom Außenraum in den Innenraum, Fouling auftritt, schlägt WO95/21687 A1 einen periodischen Wechsel der Strömungsrichtung im Innenraum vor (siehe Figur 9). Diese vermeidet zwar das Fouling, die Notwendigkeit, den Fermenter als unter Überdruck stehenden, abgeschlossenen Raum zu betreiben, um die Voraussetzung für Startling's Flow zu gewährleisten, stellt aber einen schwerwiegenden Nachteil dar. Üblicherweise werden nämlich Fermenter und Reaktoren zur Kultivierung von Zellen belüftet um dem Sauerstoffhaushalt der Zellen zu gewährleisten. Dies erfolgt so, dass sich über der gerührten Zellsuspension ein bei etwa atmosphärischem Druck gehaltener Gasraum befindet. Die Mängel der in der WO95/21687 A1 genannten Anordnung - hoher Überdruck des Reaktors und mangelnde Eignung für die Integration in konventionelle Fermentationssysteme - haben dazu geführt, dass sich die auf Starling's Flow beruhende Fouling-Kontrolle nur in externen Filtern durchgesetzt hat, die sowohl von Zellsuspension als auch von Nährlösung zwangsdurchströmt werden. Nachteilig sind hier die hohe Schwerbeanspruchung der Zellen bei Zwangsförderung und die fehlende Sauerstoffversorgung im externen Kreislauf.

DD 293 605 A5 beschreibt eine Stoffaustauscheinrichtung, die sich nicht im Reaktor befindet. Es handelt sich also um eine Anordnung bei der das Membransystem extern angeordnet ist. Darüber hinaus beschreibt DD 293 605 A5 als Stoffaustauscheinrichtung eine Packung, wobei es sich üblicherweise um ein Adsorptionsverfahren und nicht um ein Membranverfahren handelt.

DE 3914 956 A1 beschreibt ein Verfahren, bei dem abwechselnd das Innere der Kapillaren und der Reaktorraum mit Druck beaufschlagt wird. Dieses Vorgehen ist in Bioreaktoren, die mit Rührern und Belüftung ausgestattet sind, nicht praktikabel. Fast alle Fermenter werden mit Belüftung, d.h. bei atmosphärischem Druck betrieben, ein periodischer Druckaufbau in einem größeren Fermenter ist daher äußerst schwierig zu realisieren und mit der Notwendigkeit des schnellen Zuführens großer Luftmengen für den Druckaufbau im Fermenter und anschließend der Freisetzung großer Luftmengen aus dem Luftraum über dem Fermenter (Entspannung) verbunden. Dies erschwert auch die gleichmäßige Belüftung vom Boden des Fermenters.

Nachteilig in DE 3914 956 A1 ist aber auch der bei viel zu niedriger Konzentration erfolgende Austrag des Produktes, der den Aufarbeitungsaufwand wesentlich erhöht. Fig. 6 in DE 3914 956 A1 zeigt den Verlauf der Produktkonzentration im Innern der Membran, die auch nach 10 Pulsen bei deutlich weniger als 50% der Konzentration im Reaktor liegt und sich der Marke von 50% nur asymptotisch nähert, d. h. diese nicht überschreitet. Die in DE 3914 956 A1 beschriebene Kapillarpackung erlaubt keinen schnellen oder vollständigen Konzentrationsausgleich im gesamten Reaktor.

Fermentationsprozesse werden meist im Batch- oder Fedbatch-Betrieb durchgeführt. Der Anzucht der Biomasse folgt die Produkterzeugung und nach Abschluss der Fermentation die Trennung von Produktlösung und Biomasse, verbunden mit Ausschleusung und Vernichtung der Biomasse. Zur Trennung werden neben Verfahren wie Flockung mit anschließender Sedimentation oder Zentrifugation auch MF- und UF-Membranen eingesetzt. Nachteilig sind Sterilitätsprobleme bei externer Produktisolierung, vor allem aber die sehr schlechte Ausnutzung von Biomasse und Fermentern, insbesondere bei Produktinhibierung oder langer Anzuchtphase. Bei kontinuierlichen Fermentationsprozessen, die diese Nachteile nicht aufweisen, müssen die Produkte aus dem laufenden Prozess isoliert werden. Hierzu können extern oder im Reaktor angeordnete Abtrennverfahren eingesetzt werden. Manches im Batch-Verfahren einsetzbare Verfahren wie Zentrifugation kommt aber wegen der Notwendigkeit der sterilen und schonenden Biomassenrückführung in den Fermentern nicht in Frage. Membranen sind grundsätzlich einsetzbar, sind aber häufig im Reaktor angeordnet und die Biomasse verbleibt in Suspension im Fermenter. Das zentrale Problem der Abtrennung der Biomasse durch Membranen ist die bereits beschriebene Deckschichtbildung auf der Membran. Sie führt meist schnell zu einem Abfall der Filtrationsleistung, der auch durch Rückspülung, bei der erzeugtes Permeat verloren geht, nicht dauerhaft beseitigt werden kann. Bei externer Abtrennung besteht jedoch die oben erwähnte Möglichkeit der Nutzung von Starling's Flow, verbunden mit den genannten Nachteilen der Schädigung der Biomasse.

Die Wahrung der Langzeitsterilität bei der Zudosierung von Nährlösung und der Biomasserückhaltung ist bei kontinuierlichen Prozessen bisher nur schwer möglich. In der Literatur wird auf diese Problematik verwiesen, eine Lösung wurde bisher jedoch nicht gefunden, weshalb sich die kontinuierlichen Prozesse in der Praxis bisher nicht durchgesetzt haben. Im Vergleich zum Batch-Prozess weisen sie dennoch große Vorteile auf, wie die Einsparung eines zusätzlichen Prozessschrittes für die Abtrennung der Biomasse und eine deutlich kostengünstigere Betriebsweise.

Häufig ist die Isolierung von Produkten aus Bioreaktoren ein kritischer Schritt in biotechnologischen Produktionsprozessen. Dies gilt besonders für kontinuierlich betriebene Reaktoren aber auch zur Verringerung der Volumenzunahme bei Fed-Batch-Produktionen und in allen Fermentationstypen mit Produktinhibierung, zu deren Beherrschung große Suspensionsströme aus dem Reaktor abgezogen, aufgearbeitet und gegebenenfalls zurückgeführt werden müssen, was zur erheblichen Abtrennproblemen führt. Die Abtrennung der Biomasse oder der Enzyme von der Fermentationsbrühe durch Membranfiltration ist zwar ein etabliertes Verfahren, dennoch kommt es oft zu Membranfouling und Schädigung der Mikroorganismen oder Enzyme durch Scherung oder Sauerstoffmangel in externen Filtrationseinheiten. Membranfouling entsteht durch Ablagerung von bei der Filtration durch die Membran zurückgehaltenen Stoffen und führt letztendlich zu einem Abfall der Filtrationsleistung. Um dieses Problem zu verringern, kann mit Permeat rückgespült werden, wodurch es zu zusätzlichen Betriebs- und Investitionskosten kommt. Solange aber im zeitlichen Mittel ein Netto-Strom vom Reaktor über die Membran tritt, muss mit Fouling gerechnet werden.

Der vorliegenden Erfindung lag nunmehr die Aufgabe zugrunden, die zuvor genannten Probleme in einem einzigen Prozessschritt zu lösen. Insbesondere umfasst das der vorliegenden Erfindung zugrundeliegende Problem:
- den möglichst vollständigen Rückhalt partikulärer oder hochmolekularer Stoffe, z.B. Biomasse im Fermenter oder Enzyme.
- Entfernung der gelösten Produkte in möglichst hoher Konzentration aus dem Reaktor bei gleichzeitiger Vermeidung einer Deckschichtbildung auf der Membran und des daraus resultierenden Leistungsabfalls,
- sterile Zufuhr von Lösung in den Reaktor,
- Vermeidung starker Scherbelastung, vor allem bei Zellen höherer Organismen und
- Integration der Produktisolierung in konventionelle Fermenter mit dem wichtigen Spezialfall der kontinuierlichen Fermentation.

Erfindungsgemäß wird das Problem gelöst, indem eine in den Innenraum eines in einem Reaktor angeordneten Membransystems geförderte Flüssigkeit mit der im Außenraum befindlichen Produktlösung durch die porösen Membranen des Membransystems hindurch dadurch ausgetauscht wird, dass der Druck im gesamten Innenraum abwechseln über den Druck im Außenraum angehoben und unter den Druck im Außenraum abgesenkt wird.

Gegenstand der vorliegenden Erfindung ist ein Verfahren zur Durchführung von Reaktionen mit biologischem Aktivmaterial in einem Reaktor umfassend die Schritte:
a) Bereitstellen eines Reaktors umfassend
   i) mindestens einen Behälter mit einem Außenraum, in dem sich ein flüssiges Reaktionsmedium, das Produkte erzeugt, befindet, wobei das Reaktionsmedium mindestens ein biologisches Aktivmaterial, ausgewählt aus der Gruppe bestehend aus Enzymen, Zellen, Mikroorganismen und Mischungen hiervon, enthält und
   ii) mindestens einem Membransystem, das im Innern des Behälters angeordnet ist und das mindestens eine Membran und mindestens einen Innenraum aufweist, wobei mindestens eine Membran in direktem Kontakt mit dem im Behälter befindlichen Reaktionsmedium steht und den Innenraum des Membransystems von dem Außenraum abtrennt und wobei die Membran für das im Reaktionsmedium gelöste Produkt im Wesentlichen permeabel und für das biologische Aktivmaterial im Wesentlichen nicht permeabel ist und
   iii) das Membransystem, insbesondere der Innenraum des Membransystems, mit mindestens einer flüssigkeitführenden Verbindung mit mindestens einem Pulsations- und Förderungssystem verbunden ist,
b) Austausch von in dem Innenraum des Membransystems befindlicher Flüssigkeit über die Membran mit im Außenraum befindlicher Produktlösung dadurch, dass der Druck im gesamten Innenraum des Membransystems abwechselnd über den Druck im Außenraum angehoben und unter den Druck im Außenraum abgesenkt wird und
c) Ausschleusung der mit Produkt angereicherten Flüssigkeit aus dem Innenraum des Membransystems und
d) Ergänzen der aus dem Innenraum entnommenen Flüssigkeit durch produktfreie oder produktarme Flüssigkeit, zum Beispiel durch eine Substrat-oder Nährlösung oder einen produktarmen Strom aus der Aufarbeitung der entnommenen Produktlösung.

Im Rahmen der vorliegenden Erfindung wird der Begriff "Reaktor" und "Bioreaktor" synonym verwendet. Bevorzugt handelt es sich dabei um einen Fermenter oder einen enzymatischen Reaktor. Der Bioreaktor umfasst einen Behälter (Reaktionsbehälter), in dem sich das flüssige Reaktionsmedium, das üblicherweise biochemisch herstellbare bzw. biotechnologisch herstellbare Produkte erzeugt und mindestens ein biologisches Aktivmaterial, ausgewählt aus der Gruppe bestehend aus Enzymen, Zellen, Mikroorganismen und Mischungen hiervon, enthält. Der Reaktionsbehälter des Reaktors weist wenigstens einen Außenraum, in dem sich das flüssige Reaktionsmedium befindet sowie zusätzlich ein Membransystem auf. Gemäß dem Verfahren der vorliegenden Erfindung wird die in den Innenraum des Membransystems geförderter Flüssigkeit mit der im Außenraum befindlichen Produktlösung durch die, vorzugsweise poröse, Membran des Membransystems hindurch dadurch ausgetauscht, dass der Druck im gesamten Innenraum abwechselnd über den Druck im Außenraum angehoben und unter den Druck im Außenraum abgesenkt wird.

Bevorzugt im Rahmen des vorliegenden erfindungsgemäßen Verfahrens erfolgt die Pulsation ausschließlich durch Druckänderungen im Innenraum des Membransystems.

In einer bevorzugten Ausführungsform ist die Membran eine Ultra- oder Mikrofiltrationsmembran, die bevorzugt mittels geeigneter Halterungs-, Verbindungs-und Dichtungselemente so in den Behälter des Reaktors eingesetzt werden kann, dass sie einen oder mehrere Räume, im Folgenden "Innenraum" genannt, von dem eigentlichen Behälter, dem "Außenraum", abtrennen, indem sich das flüssige Reaktionsmedium, vorzugsweise die Fermentersuspension oder die Enzymlösung, befindet.

Bevorzugt wird gemäß der vorliegenden Erfindung ein großer Teil der mit dem Außenraum in Kontakt stehenden Membranfläche, besonders bevorzugt mindestens 50 %, insbesondere mindestens 60 %, insbesondere mindestens 80 % der Fläche, im Wirkungsbereich des Rührers und/oder der Belüftung angeordnet. Dadurch kann die Produktkonzentration im Innern des Membransystems weiter gesteigert werden.

Der Druck im gesamten Innenraum wird bevorzugt in regelmäßigen Zeitintervallen durch ein Pulsations- und Fördersystem so über den Druck im Außenraum des Behälters angehoben und dann wieder unter diesen abgesenkt, dass ein periodisch die Richtung wechselnder Filtratstrom durch die Membranen tritt. Der periodische Flüssigkeitsaustausch erfolgt vorzugsweise so, dass im zeitlichen Mittel der vom Außenraum über die Membran in den Innenraum tretende Volumenstrom höchstens so groß ist wie der Strom in umgekehrter Richtung. Gemäß einer bevorzugten Ausführungsform des erfindungsgemäßen Verfahrens erfolgt ebenfalls in periodischen Abständen die Zufuhr von frischer Lösung in den Innenraum des Membransystems und die Entnahme von produktreicher Flüssigkeit aus dem Innenraum des Membransystems. Hierzu besitzt der Innenraum mindestens eine gegenüber dem Außenraum flüssigkeitführende, vorzugsweise abgeschlossene, aus dem Behälter führende Verbindung mit Elementen des außerhalb des Reaktors befindlichen Pulsations- und Fördersystems. Üblicherweise handelt es sich bei der flüssigkeitführenden Verbindung um einen Schlauch. Bevorzugt wird der Austausch in Schritt b) durch eine periodisch die Richtung wechselnde Durchströmung der Membran und des Innenraums durch die Aufprägung eines periodisch wechselnden Drucks im Innenraum erreicht wird, wobei der Druck im gesamten Innenraum abwechselnd über den Druck im Außenraum angehoben und unter diesen abgesenkt wird und die Druckniveaus und Taktzeiten und die Fläche der Membran entsprechend der Membranpermeabilität so gewählt werden, dass einerseits ein zur Ausschleusung des Produktes ausreichender Flüssigkeitsaustausch über die Membran stattfindet, andererseits im längerfristigen Mittel der gewünschte Verlauf des Füllstandes im Reaktor erreicht wird.

Besonders bevorzugt erfolgt der Austausch in Schritt b) durch eine periodisch die Richtung wechselnde Durchströmung der Membran durch abwechselnde, taktweise Zwangsförderung von Flüssigkeit in den Innenraum des Membransystems hinein und von mit Produkt angereicherter Flüssigkeit aus dem Innenraum heraus, wobei der Druck im gesamten Innenraum abwechselnd über den Druck im Außenraum angehoben und unter diesen abgesenkt wird und die Taktvolumina und Taktzeiten und die Membranfläche entsprechend der Membranpermeabilität so gewählt werden, dass einerseits ein zur Ausschleusung des Produktes ausreichender Flüssigkeitsaustausch über die Membran stattfindet, andererseits im längerfristigen Mittel der gewünschte Verlauf des Füllstandes im Reaktor erreicht wird. Die Membranen des Membransystems sind so ausgelegt, dass sie für das im Reaktionsmedium gelöste Produkt im Wesentlichen permeabel und für das biologische Aktivmaterial im wesentlichen nicht permeabel sind. Die Porengröße der Membran wird vorzugsweise so gewählt, dass die gelösten Produkte und die Nährlösungskomponenten die Membran passieren können, das Aktivmaterial, also beispielsweise Zellen oder Enzyme oder Mikroorganismen, die im Reaktor die Produkte erzeugen, jedoch zurückgehalten werden. Zur Rückhaltung von Zellen oder Mikroorganismen verwendet eine bevorzugte Ausführungsform des Verfahrens als Membran Mikrosiebe. Mikrosiebe zeichnen sich durch besonders gleichmäßige Porengrößen und geringen Strömungswiderstand aus. Bevorzugt werden als Membran ein oder mehrere Mikrosiebe verwendet, vorzugsweise mit einem maximalen Porendurchmesser oder einer maximalen Schlitzweite von 10 Mikrometern. Bei der über den Innenraum in den Außenraum zugeführten Flüssigkeit handelt es sich bevorzugt um ein Nährmedium, es ist aber auch möglich, eine aus der Aufarbeitung der Produkte zurückgeführte und gegebenenfalls mit Nährmedium angereicherte Lösung oder eine beliebige andere Flüssigkeit einzusetzen. In einer Ausführungsform des Verfahrens wird eine Suspension von funktionalisierten Partikeln zugeführt, bei denen es sich um Partikel mit Ionenaustauscherfunktion, Affinitätsfunktionalität und spezifischer Adsorbensfunktionalität oder einer Bindefunktionalität für bestimmte Stoffgruppen oder Einzelstoffe handeln kann.

Besonders bevorzugt ist die in Schritt b) im Innenraum des Membransystems befindliche Flüssigkeit eine Suspension von Adsorbenspartikeln ist, wobei die für die Membran für die Adsorbenspartikel im Wesentlichen nicht permeabel sind und wobei die Adsorbenspartikel das Produkt und/oder ein inhibierendes Nebenprodukt binden und vorzugsweise die beladenen Partikel nach der Beladung ausgeschleust und ganz oder teilweise regeneriert und dann wieder eingesetzt werden. Die Zugabe von Adsorbenspartikeln in die zugeführte Lösung erlaubt eine integrierte Aufarbeitung. Funktionalisierte Partikel werden im Innenraum des Membransystems beladen und extern regeneriert. Dies kann durch pH-, Ionenstärke- oder Lösemittelwechsel erfolgen. Nach Abtrennung des Regenerats und gegebenenfalls einem Waschschritt können die funktionalisierten Partikel wieder zur Aufnahme frischen Produktes eingesetzt werden. Im Vergleich zu klassischen chromatografischen Methoden ist nur eine kleine Menge des häufig sehr teuren funktionalisierten Adsorbermaterials erforderlich. Dieser Vorteil kann zum Beispiel bei der Produktion monoklonaler Antikörper genutzt werden.

Das erfindungsgemäße Verfahren besitzt gegenüber konventioneller Membranfiltration folgende Vorteile:
- Vermeidung von Membranfouling.
   Bei konventioneller Membranfiltration reichern sich auf der Membran die zurückgehaltenen Komponenten an und bilden eine Deckschicht, die die Filtration behindert und oft zum Erliegen bringt. Häufig tritt auch Anhaften und Wachstum antransportierter Mikroorganismen auf der Membran auf. Diese als Membranfouling bezeichneten Phänomene stellen die wichtigste Einschränkung der Einsetzbarkeit von Membranverfahren in der Bioverfahrenstechnik dar.
   Das erfindungsgemäße Verfahren vermeidet diese Probleme, da im längerfristigen Mittel kein Netto-Flüssigkeitstransport aus dem Fermenter durch die Membran erfolgt, sich also auch keine Inhaltsstoffe des Fermenters dauerhaft an der Membran anreichern. Kurzzeitig blockierte Poren werden regelmäßig durch den Flüssigkeitsrücktransport frei gespült, was den Abfall der Filtrationsleistung verhindert.
- Beherrschung von Produktinhibierung
   Produktinhibierung erfordert Abzug und Aufarbeitung großer Ströme an Inhalt des Reaktionsbehälters des Reaktors. Das erfindungsgemäße Verfahren erlaubt dies ohne die Nachteile des Membranfoulings. Nach Abtrennung des Produktes in darauf folgenden Aufbereitungsschritten, die durch die Membranfiltration wesentlich erleichtert werden, wird der Hauptteil der Flüssigkeit über den Innenraum wieder in den Fermenter oder Enzymreaktor zurückgeführt. Dies minimiert Produktverluste.
- Schonende Aufarbeitung
   Grundsätzlich handelt es sich beim erfindungsgemäßen Verfahren um eine besonders schonende Behandlung der Mikroorganismen oder Zellen. Auf der Seite der Fermentationsbrühe oder Reaktionslösung ist keine mit Scherwirkung verbundene Druckänderung erforderlich. Es tritt auch keine Sauerstoffabreicherung auf, die bei externer Aufarbeitung ein Problem darstellt.
- Einhaltung der Wasserbilanz, gleichmäßiger Betrieb bei Konti-Fermentation Bei Wahl eines Nährmediums als zugeführte Flüssigkeit kann bis auf den Abzug von Suspension zur Korrektur des Biomassenwachstums auf jegliche sonstige Wasserzu- oder -Abfuhr verzichtet werden. Es treten keine nennenswerten Konzentrationsschwankungen auf, die einen kontinuierlichen Fermentationsbetrieb stören. Die Regelung des Flüssigkeitsstands im Reaktor ist problemlos möglich.
- Sterilität, unterbrechungsfreier Betrieb
   Der stationäre Betrieb erlaubt einen Verzicht auf Öffnen des Fermenters und die Membran erleichtert die Einhaltung der Sterilität. Im Vergleich zum Einsatz konventioneller Membranlagen erlaubt das verringerte Fouling wesentlich längere Standzeiten ohne Eingriff, was Anlagenverfügbarkeit und -Produktivität erhöht.
- Längere produktive Phase bei Fed-Batch-Fermentationen
   Bei Fed-Batch-Fermentationen ist die produktive Phase durch die Zunahme des im Reaktor befindlichen Flüssigkeitsvolumens begrenzt. Diese Einschränkung entfällt beim erfindungsgemäßen Verfahren.
- Senkung der benötigten Mengen an Adsorbentien in der Aufarbeitung

Es ist möglich, den in Schritt b) mit dem Austausch von Flüssigkeit zwischen Innenraum und Außenraum verbundenen Flüssigkeits-Hin- und Rücktransport über die Membran hinweg gleichzeitig jeweils zur Zuführung von Flüssigkeit und zum Abtransport von Produktlösung zu nutzen. Der An- und Abtransport kann aber auch in separaten Zufuhr und Entnahmetakten d und c erfolgen. In beiden Varianten wird ein kontinuierlicher Betrieb mit einem kontinuierlichen Austausch von einerseits Produkt und andererseits Substrat, Edukt oder Nährlösung unter sterilen Bedingungen ermöglicht.

In einer bevorzugten Ausgestaltung ist das im zeitlichen Mittel aus dem Außenraum in den Innenraum durch die Membran tretende Flüssigkeitsvolumen höchstens ebenso groß ist wie das im zeitlichen Mittel aus dem Innenraum in den Außenraum durch die Membran tretende Flüssigkeitsvolumen.

Wichtig für biotechnologisch durchgeführte Verfahren ist die stabile Zufuhr von Edukten und Substraten in das Reaktionsmedium. Bevorzugt enthält daher die über den Innenraum zugeführte Flüssigkeit mindestens ein zur Erzeugung des/der Produkt(e) in dem Reaktionsmedium benötigtes Edukt oder Substrat.

In einer weiteren Ausgestaltung des Verfahrens der vorliegenden Erfindung stammt die über den Innenraum zugeführte Flüssigkeit zumindest teilweise aus einer Aufarbeitung der in Schritt c) ausgeschleusten Flüssigkeit und ist bezüglich der Produktkonzentration gegenüber der ausgeschleusten, produktangereicherten Flüssigkeit abgereichert.

In einer weiteren bevorzugten Ausgestaltung des Verfahrens schließt sich an die Ausschleusung der mit Produkt angereicherten Flüssigkeit aus dem Innenraum des Membransystems in Schritt c) eine Produktaufarbeitung an, vorzugsweise durch Verwendung ein oder mehrerer weiterer Membranverfahren, wobei weiter bevorzugt die an Produkt abgereicherte Flüssigkeit ganz oder teilweise über den Innenraum des Membransystems in Schritt b) dem Außenraum des Reaktorbehälters zugeführt wird.

Besonders bevorzugt handelt es sich bei dem zur Produktaufarbeitung eingesetzten Membranverfahren um eine Nanofiltration oder Umkehrosmose, deren Permeat vorzugsweise ganz oder teilweise über den Innenraum des Membransystems in Schritt b) dem Außenraum des Reaktorbehälters zugeführt wird oder dass es sich bei der Produktaufarbeitung um eine organophile Pervaporation handelt, deren Retentat vorzugsweise über den Innenraum des Membransystems in Schritt b) dem Außenraum des Reaktorbehälters zugeführt wird.

Bevorzugt wird als Produktaufarbeitungsverfahren eine Elektrodialyse verwendet wird, deren Diluate vorzugsweise ganz oder teilweise über den Innenraum des Membransystems in Schritt b) dem Außenraum des Reaktorbehälters zugeführt wird oder das als eines der Produktaufarbeitungsverfahren eine Elektrodialyse mit bipolaren Membranen verwendet wird, deren an Produkt abgereicherte Flüssigkeit (Ausgangsstrom) vorzugsweise ganz oder teilweise über den Innenraum des Membransystems in Schritt b) dem Außenraum des Reaktorbehälters zugeführt wird.

In einer bevorzugten Ausgestaltung des erfindungsgemäßen Verfahrens wird der Innenraum des Membransystems periodisch belüftet und von Flüssigkeit entleert und die entnommene Flüssigkeit entsprechend ihrer Zusammensetzung dem Innenraum später wieder zugeführt oder mit der entnommenen Produktlösung ausgeschleust. Hierdurch kann eine weitere Erhöhung der entnommenen Produktkonzentration ohne unerwünschte Erhöhung des ausgetauschten Volumenstroms, d.h. ohne weitere Vergrößerung der Membranfläche erreicht werden. Hierzu wird vorteilhaft nach einem Entnahmetakt das Innenraumvolumen durch Belüftung mit, vorzugsweise über ein Sterilfilter, zugeführter Druckluft entleert, wobei die dabei entnommene Flüssigkeit dem Entnahmevolumen (Produktstrom zur Aufarbeitung) zugeführt wird, während die aus dem Innenraum nach einem Zuführtakt entnommene Flüssigkeit in analoger Weise zurück in den Vorratsbehälter der Flüssigkeitszufuhr gedrückt wird.

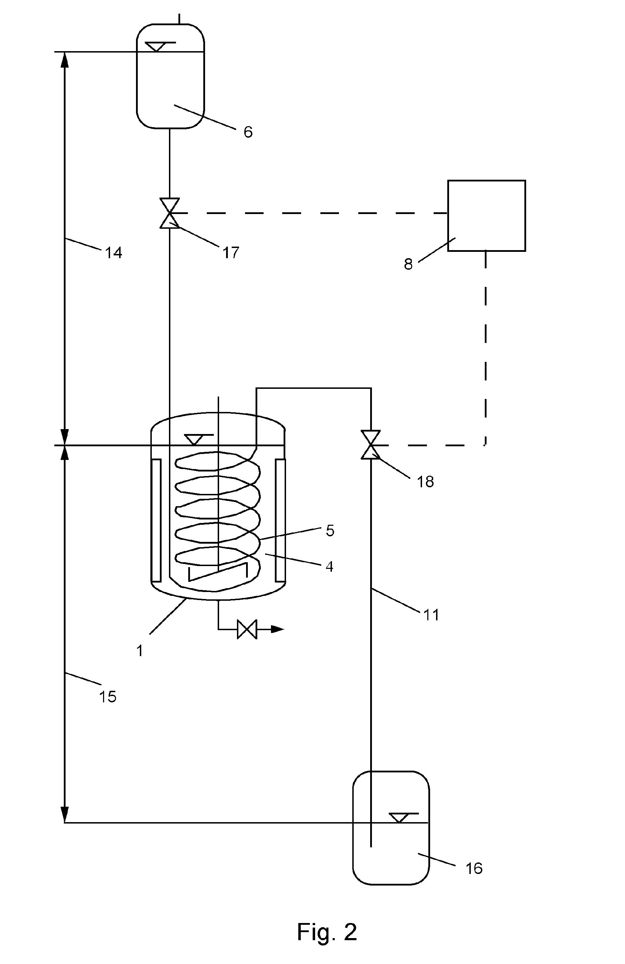

Es wurde gefunden, dass eine Produktkonzentration von mindestens 50% der Konzentration im Außenraum, in dem sich das flüssige Reaktionsmedium befindet (Fermenter), im Innern des Membransystems nur erreicht werden kann, wenn pro Periode, d.h. zwischen Beginn eines Zufuhrtaktes und dem Ende eines Entnahmetaktes mehr als das Doppelte des gesamten Innenraumvolumens des Membransystems über die Membran ausgetauscht wird. Im Rahmen der vorliegenden Erfindung setzt sich das Innenraumvolumen des Membransystems zusammen aus den Volumina der Membranen, inklusive des Porenanteils der Wände und den Volumina der Zuleitungen und Verbindungen zwischen den Membranen und den den Innenraum absperrenden Ventilen (vgl. Fig. 2, Fig. 4 und Fig. 5). Es ist zwar möglich, das pro Periode über die Membran ausgetauschte Volumen in mehrere Austauschtakte aufzuteilen, wodurch das Fouling der Membran verringert wird, eine solche Aufteilung verringert jedoch die Produktkonzentration bei der Produktentnahme. Die Anzahl der aufeinander folgenden Austauschtakte innerhalb einer Periode sollte daher auf zwei bis drei begrenzt bleiben, um nicht die insgesamt benötigte Membranfläche zu stark ansteigen zu lassen.

Vorteilhafterweise sollte zur Erzielung der höchsten Produktkonzentration für ein bestimmtes pro Periode ausgetauschtes Volumen sollte auf eine Aufteilung dieses Volumens in mehrere Austauschtakte ganz verzichtet werden.

Zur Erzielung deutlich höherer Produktkonzentration als 50% der Konzentration im Fermenter ist es erforderlich, das pro Periode insgesamt über die Membran ausgetauschte Volumen zu steigern. Mit Austausch von drei Innenraumvolumina pro Periode und vorzugsweise maximal zwei zwischengeschalteten Austauschtakten erzielt man 65 bis 70% der Produktkonzentration des Fermenters in der Entnahme und zur Erzielung von 80 bis 85% müssen vorteilhafterweise mindestens 5 Innenraumvolumina ausgetauscht werden.

In einer besonderen Ausgestaltung erfolgt die Taktung der zur Förderung benutzten Ventile und Pumpen so, dass nicht in jedem Takt Produktlösung entnommen wird, sondern in einem Teil der Takte nur Flüssigkeit zwischen Innenraum und Außenraum ausgetauscht wird.

Besonders bevorzugt ist das erfindungsgemäße Verfahren für die Herstellung von Alkoholen geeignet. Bevorzugt wird als Produkt ein Alkohol, vorzugsweise ein aliphatischer Alkohol, insbesondere Ethanol oder Butanol erzeugt.

Das Verfahren kann aber auch zur Herstellung organischer Säuren verwendet werden. Bevorzugt wird daher als Produkt eine organische Säure, vorzugsweise Itaconsäure erzeugt.

Aufgrund der hervorragenden Eigenschaften hinsichtlich der Stabilität bei der Zufuhr von Substraten und Edukten eignet sich das Verfahren auch insbesondere für die Herstellung monoklonaler Antikörper. Bevorzugt wird daher als Produkt ein monoklonaler Antikörper erzeugt.

Ein weiterer Gegenstand der Erfindung ist ein Bioreaktor umfassend
a) mindestens einen Behälter mit einem Außenraum zur Aufnahme eines flüssigen Reaktionsmediums,
b) mindestens ein Membransystem, das im Innern des Behälters angeordnet ist und das mindestens eine Membran und mindestens einen Innenraum aufweist, wobei mindestens eine Membran den Innenraum des Membransystems von dem Außenraum abtrennt und wobei das Membransystem mit mindestens einem Pulsations- und Fördersystem verbunden ist.

Die bevorzugten Ausgestaltungen, die zuvor im Zusammenhang mit dem erfindungsgemäßen Verfahren genannt wurden, gelten ebenso für den Bioreaktor.

Bevorzugt weist der Bioreaktor zusätzlich ein Produktaufarbeitungssystem auf.

Die Reaktoren sind vorteilhafterweise mit Belüftungs- und/oder Rührvorrichtungen ausgestattet. Die Belüftungsvorrichtung hat die Aufgabe den Druck im Außenraum (4), in dem sich das Reaktionsmedium befindet vorzugsweise im Wesentlichen konstant zu halten. Es ist für die Durchführung des erfindungsgemäßen Verfahrens von Vorteil, wenn Druckschwankungen möglichst ausgeglichen werden können und wenn der Druck im Außenraum vorzugsweise während des gesamten Verfahrens dem Atmosphärendruck entspricht.
Die Reaktoren können darüber hinaus Rührvorrichtungen aufweisen, die das Reaktionsmedium durchmischen. Geeignete Rührvorrichtungen sind dem Fachmann geläufig.

Bevorzugt ist das Pulsations- und Fördersystem über mindestens eine gegenüber dem Außenraum flüssigkeitsdichte, flüssigkeitführende Verbindung mit dem Innenraum des Membransystems verbunden.

In einer bevorzugten Ausgestaltung des Bioreaktors ist das Membransystem ausgewählt aus der Gruppe bestehend aus Kapillarmembranen und Rohrmembranen.

Die Erzielung hoher Konzentrationen durch Steigerung des Austauschvolumens wird durch Verwendung neuartiger Hochleistungsmembranen, sogenannter Mikrosiebe, die z.B. aus Siliziumscheiben hergestellt werden können, wesentlich erleichtert. Hiermit lassen sich leicht auch in größeren Reaktoren, in denen es schwieriger ist, die benötigte Membranfläche strömungsgünstig anzubringen, ausreichend große Austauschvolumina pro Periode realisieren, um hohe Produktkonzentrationen zu erreichen.

Bevorzugt ist die Membran des Membransystems ein Mikrosieb.

Ein weiterer Gegenstand der vorliegenden Erfindung ist die Verwendung des erfindungsgemäßen Bioreaktors für biochemische Reaktionen, insbesondere Fermentationen, enzymatischer Umsetzungen oder der Produktion monoklonaler Antikörper.

Die das erfindungsgemäße Verfahren kennzeichnenden Merkmale und Anlagenkomponenten werden nachfolgend an Hand von Prinzipskizzen erläutert. Es zeigen:

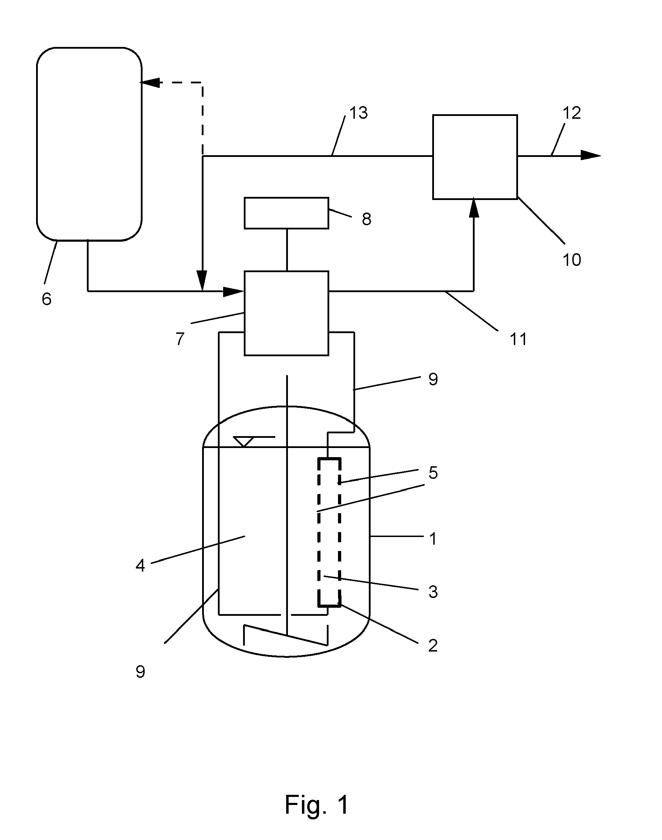

**Fig. 1** einen erfindungsgemäßen Bioreaktor mit den Hauptanlagenkomponenten und der Einbindung einer Produktaufarbeitung in das Verfahren,
**Fig. 2** eine Variante, in der die Flüssigkeitsförderung und der Flüssigkeitsaustausch durch die Membran durch die Schwerkraft bewirkt und nur eine einzelne Kapillare als Membran verwendet wird,
**Fig. 3** eine Variante, die nur eine Verbindung zwischen Membransystem und Pulsations- und Fördersystem und einen Hubkolben als Förderorgan aufweist,
**Fig. 4** eine bevorzugte Variante, die zwei Verbindungen zwischen Membransystem und Pulsations- und Fördersystem und zwei Pumpen aufweist,
**Fig. 5** eine bevorzugte Variante, die eine besonders hohe Produktkonzentration im abgezogenen Produktstrom erlaubt,
**Fig. 6** eine schematische perspektivische Ansichten einer Anordnung von Kapillar- und Rohrmembranen in größeren Reaktoren,
**Fig. 7a** und **Fig. b** schematische Schnittbilder einer Anordnung von Mikrosieben in einem Reaktor
**Fig. 8** ein Schema eines Regenerationssystems für Adsorbentien und
**Fig. 9** die Strömungs- und Druckverhältnisse bei Starlings Flow (nicht erfindungsgemäß)

Eine erfindungsgemäß bevorzugte Ausgestaltung ist in Fig**.** 1 gezeigt und wird nachfolgend erläutert. Die Anlage besteht aus mindestens einem Reaktor **1**, aus einem Membransystem **2**, das einen Innenraum **3** gegenüber dem Außenraum **4** abtrennt und eine oder mehrere Membranen **5** umfasst, die innerhalb des Behälters des Reaktors **1** angeordnet sind und mit ihrer Außenseite die im Außenraum **4** befindliche Produktlösung oder -Suspension direkt berühren, einem Vorratsbehälter **6** für die zugeführte Lösung, einem außerhalb des Reaktors angeordneten Pulsations- und Fördersystem **7**, einer damit verbundenen Steuereinheit **8**, gegebenenfalls mit selbiger verbundenen Messeinrichtungen, sowie geeigneten Verbindungsleitungen **9**, die einen oder mehrere Ein- beziehungsweise Ausgänge des Membransystems **2** mit dem Pulsations- und Fördersystem **7** verbinden. Darüber hinaus kann ein Aufarbeitungssystem **10** der Produktlösung **11** integriert werden. Hierbei kann es sich um Adsorption, um Membranverfahren wie Umkehrosmose, Nanofiltration, Pervaporation oder Elektrodialyse, um Extraktion, Kristallisation oder Destillation oder deren Kombination handeln. Das Aufarbeitungssystem **10** erzeugt aus der entnommenen Produktlösung **11** produktreiche Ströme **12**, die weiterverarbeitet oder entnommen werden und produktarme Ströme **13**, die wieder über das Pulsations- und Fördersystem **7**, gegebenenfalls nach Anreicherung mit Nährmedium in den Reaktor **1** zurückgeführt werden können.

### Membransystem

Das Membransystem umfasst vorzugsweise eine oder mehrere im Reaktor angeordneten UF- oder MF-Membranen, welche in Untereinheiten mit gemeinsamer Zu- und Ableitung zusammengefasst sein können und auf der Außenseite direkten Kontakt mit der im Außenraum befindlichen Suspension/Lösung besitzen. Die Membranen können aus polymerem oder anorganischem Material bestehen. Die Porengröße der verwendeten Membranen ist so bemessen, dass die gelösten Produkte und die Nährlösungskomponenten die Membran passieren können, Zellen oder Enzyme, die im Reaktor die Produkte erzeugen, jedoch zurückgehalten werden.

Der Innenraum des Membransystems wird durch das Pulsations- und Fördersystem über mindestens eine gegenüber dem Außenraum abgeschlossene Verbindung mit zugeführter Flüssigkeit oder Adsorbenssuspension versorgt. Die Entnahme der produktreichen Lösung oder Suspension erfolgt durch die genannte oder weitere gegenüber dem Außenraum abgeschlossene Verbindungen zum Innenraum.

Das Volumen des Innenraums inklusive der Zu- und Ableitungen sollte zur Erreichung einer möglichst hohen Rückspülfrequenz möglichst klein sein. Weiter ist es von Vorteil, den Innenraum so zu gestalten, dass er möglichst gleichmäßig durchströmt wird und eine möglichst vollständige Entleerung zulässt. Andererseits muss der Druckverlust bei der Durchströmung des Innenraums möglichst klein im Vergleich zu den Druckverlusten in der Membran sein um eine gleichmäßige Permeation der Membran in den Druck und Saugtakten zu gewährleisten. Die Anordnung im Reaktor kann in vielfältiger Weise erfolgen und zwar möglichst so, dass die gesamte äußere Membranfläche intensiv von der dort befindlichen Suspension oder Lösung umspült wird.

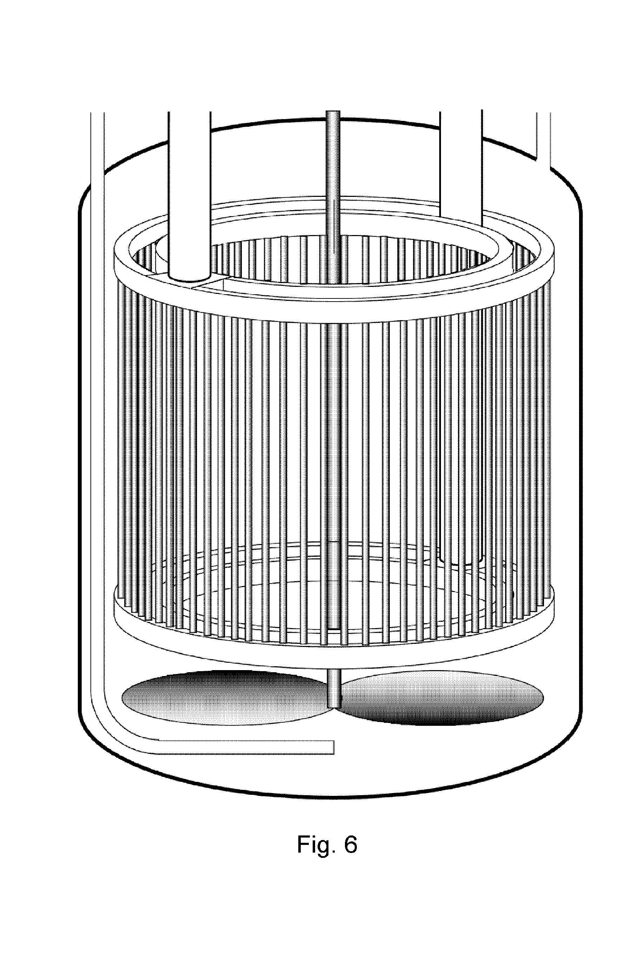

Diese Bedingungen werden zum Beispiel von parallel durchströmten polymeren Kapillarmembranen mit Innendurchmessern von vorzugsweise unter 2 mm und Längen von vorzugsweise unter 2 m erfüllt, die unterschiedlich angeordnet sein können, für Laborreaktoren zum Beispiel als Einzelkapillare in Form einer Helix, wie in **Fig. 2** gezeigt, für etwas größere Reaktoren zum Beispiel senkrecht parallel angeordnet mit der Kontur eines oder mehrerer Kreiszylinder wie in **Fig. 6** gezeigt, oder in Form von radial im Reaktor ausgerichteten senkrechten Ebenen. Die Membranen können innerhalb der Ebenen entweder senkrecht oder waagerecht ausgerichtet sein. Bei hoher Rührintensität und Verwendung wenig steifer Membranen empfehlen sich Stützelemente einzubauen. Bei Verwendung von Rohrmembranen, zum Beispiel aus Keramik, sind wegen des geringeren Druckverlusts längere Strömungswege und Hintereinanderschaltung mehrerer Rohre möglich.

Wenn große Membranflächen erforderlich sind, ist die Verwendung einer Anzahl senkrecht stehender Membranbündel vorteilhaft, die vorzugsweise an beiden Enden gemeinsame Zu-, bzw. Ableitungen besitzen. Diese Anordnung ist von den in der Abwasserreinigung verwendeten Membran-Bio-Reaktoren bekannt. Durch zwischen die Kapillaren am unteren Ende der Bündel eingeleitete Luft muss dabei ein Austausch der Flüssigkeit im Bündel mit dem Rest des Reaktors erzielt werden.

Besonders kleine Membranflächen und kleine Innenräume werden bei Verwendung so genannter Mikrosiebe als Membranen erreicht. Diese bestehen zum Beispiel aus mit Siliziumnitrid beschichteten Siliziumscheiben, in die fotolithografisch sehr gleichmäßige Poren mit einem Durchmesser um oder unter einem Mikrometer geätzt sind. Es sind aber auch polymere Mikrosiebe bekannt. Wegen ihrer Porengröße sind Mikrosiebe nicht zum Rückhalt von Enzymen geeignet.

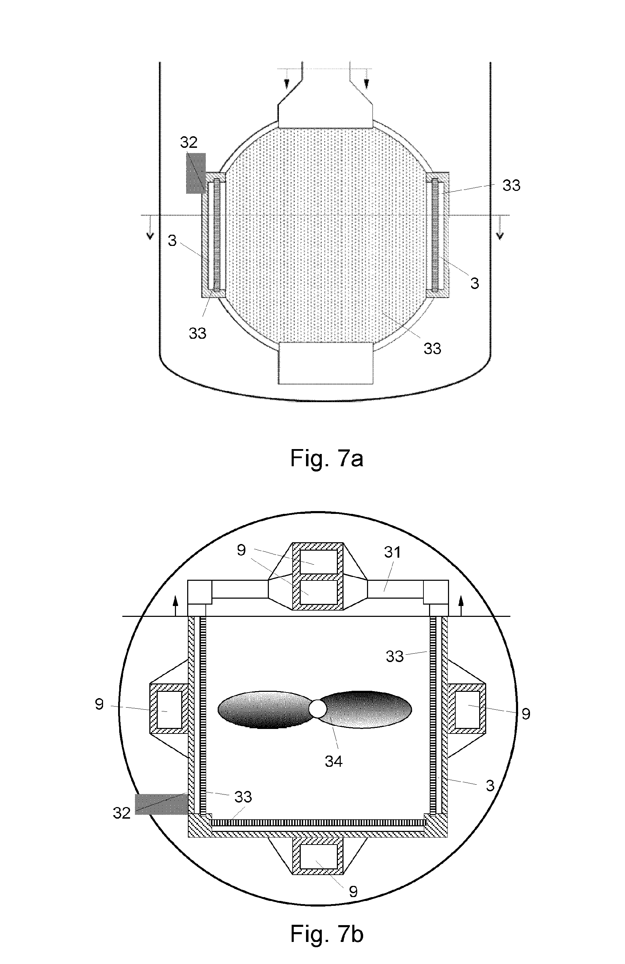

Die Anordnung der Membranen erfolgt vorzugsweise in Zonen des Behälters mit hoher Turbulenz, da hier eine besonders gute Umspülung der Außenseite der Membran erforderlich ist, also zum Beispiel senkrecht stehend in der Nähe des Rührerumfangs, wie in **Fig. 7a** und **Fig. 7b** gezeigt. Es können Plattenmodule **31** eingesetzt werden, die nur je ein vorzugsweise dem Rührer **34** zugewandtes Mikrosieb **33** enthalten und deren Innenraum **2** von der Membran und einer in geringem Abstand davon befindlichen geschlossenen Rückwand **32** und den Zu-und Ableitungen gebildet wird. Die Anordnung in **Fig. 7a** und **Fig. 7b** enthält je eine Zu- und Ableitung pro Modul, die innerhalb oder außerhalb des Reaktors vereinigt werden können, also insgesamt acht Verbindungsleitungen **9**, von denen fünf sichtbar sind. Es sind aber auch Module verwendbar, die je zwei parallele, durch einen Teil des Innenraums getrennte Mikrosiebe enthalten, in dem sich ein Abstandshalter, ein so genannter Spacer befinden kann.

Grundsätzlich sollten die verwendeten Membranen hydrophil sein, das heißt durch wässrige Lösungen benetzbar. Dies ist erforderlich, damit beim meist nicht zu vermeidenden Ausgasen von gelösten Gasen und beim später in Variante 3 beschriebenen Entleeren des Innenraums die Membran nicht durch Gas blockiert wird.

### Pulsations- und Fördersystem

Das Pulsations- und Fördersystem besteht aus Förderaggregaten wie Pumpen oder Kolben sowie Ventilen und hat die Aufgabe, einerseits die Förderung durch den Innenraum zu gewährleisten, andererseits einen periodisch die Richtung wechselnden Lösungsstrom über die Membran hinweg zu erzeugen.

Grundsätzlich lässt sich der gewünschte Flüssigkeitsaustausch über die Membran hinweg durch zwei Methoden erreichen:

Vorgabe eines definierten, periodisch schwankenden Druckverlaufs auf der dem Innenraum zugewandten Seite der Membran.

Diese Variante wird bei Schwerkraftförderung gewählt. **Fig. 2** zeigt eine geeignete Anordnung. Die Höhendifferenz **14** der Flüssigkeitsspiegel im Vorratsbehälter **6** und im Reaktor **1** bewirkt zunächst bei geöffnetem Ventil **17** und geschlossenem Ventil **18** die Förderung von Flüssigkeit vom Vorratsbehälter **6** in den Innenraum des Membransystems und von dort durch die Membran **5** in den Außenraum **4**. Dann wird Ventil **17** geschlossen und Ventil **18** geöffnet und die Höhendifferenz **15** der Flüssigkeitsspiegel von Reaktor **1** und Produktbehälter **16** bewirkt eine Förderung von Flüssigkeit aus dem Außenraum **4** durch die Membran **5** in den Innenraum und von dort in den Produktbehälter **16.** Nach Öffnung von Ventil **17** und Schließung von Ventil **18** wiederholen sich diese Vorgänge periodisch.

Zur Erreichung hoher Produktkonzentrationen im entnommenen Strom **11** sollte das pro Periode durch die Membran **5** und aus dem Innenraum gesaugte Volumen möglichst groß gegenüber dem Volumen des Innenraums sein. Da andererseits kurze Periodendauer zu besserer Rückspülung der Membran **5** führt, kann die Periodendauer im Betrieb optimiert werden. Durch Regelung des Verhältnisses der Dauer der beiden Takte einer Periode wird durch die Steuereinheit **8** der gewünschte mittelfristige Verlauf des Volumens im Reaktor **1** eingestellt.

Abwechselnde Zwangsförderung einer definierten Lösungs- oder Suspensionsmenge in den Innenraum des Membransystems sowie aus diesem heraus.

In dieser bevorzugten Ausführung des Verfahrens stellt sich ein periodisch wechselnder Druckverlauf durch abwechselnde Zwangsförderung in den abgeschlossenen Innenraum hinein und aus diesem heraus in Kombination mit dem Filtrationswiderstand der Membran von selbst ein. Es sind zahlreiche erfindungsgemäße Varianten der taktweisen Zwangsförderung denkbar, die unterschiedliche Kombinationen von Förderorganen, Ventilen und Behältern umfassen, von denen vier bevorzugte im Folgenden erläutert werden.

### Variante 1

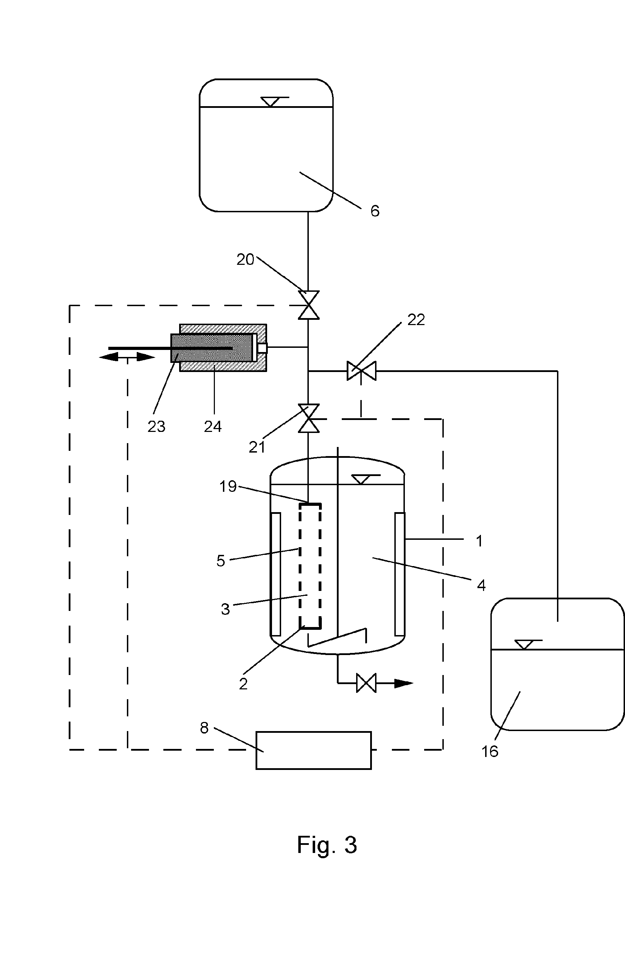

In der einfachsten, in **Fig. 3** gezeigten Ausführung besitzt der Innenraum **3** nur eine Öffnung **19,** die als Ein- und Ausgang dient. Als Pulsations- und Fördersystem wird eine Kombination von drei durch die Steuereinheit **8** geeignet getakteten Ventilen **20, 21** und **22** und einem ebenfalls getakteten Förderaggregat mit umkehrbarer Förderrichtung, zum Beispiel einer Schlauchpumpe oder wie in **Fig. 3** gezeigt, einem in einem Zylinder **24** bewegbaren Kolben **23** verwendet. Das Hubvolumen des Kolbens **23** sollte vorzugsweise größer als der Innenraum **3** des Membransystems **2** sein. Die Funktion wird im Folgenden erläutert.

Ausgangspunkt ist der in **Fig. 3** gezeigte Zustand, in dem sämtliche Ventile geschlossen und der Kolben **23** ganz in den Zylinder **24** eingefahren sind.

Schritt 1: Ventil **20** wird geöffnet und der Kolben **23** wird ausgefahren. Hierbei strömt frische Lösung aus dem Vorratsbehälter **6** in den Zylinder **24**.

Schritt 2: Ventil **20** wird geschlossen, Ventil **21** wird geöffnet und der Kolben **23** fährt in den Zylinder 24 hinein. Hierbei wird die im Zylinder **24** befindliche Lösung in den Innenraum **3** des Membransystems **2** und zusammen mit der dort befindlichen Flüssigkeit durch die Membran **5** in den Außenraum **4** gefördert. Schritt 3: Bei unveränderter Ventilstellung fährt der Kolben **23** aus dem Zylinder **24** heraus. Hierbei wird Produktlösung aus dem Außenraum **4** durch die Membran **5** in den Innenraum **3** und von dort in den Zylinder **24** gesaugt.

Schritte 2 und 3 können nun beliebig oft wiederholt werden. Ziel der Wiederholung ist eine möglichst vollständige Angleichung der Produktkonzentration im Zylinder **24** an die des Außenraums **4**. Je größer das Verhältnis des Hubvolumens des Kolbens **23** zum Volumen des Innenraums **3** ist, desto weniger Wiederholungen der Schritte 2 und 3 sind zur Erzielung einer ausreichend hohen Produktkonzentration in der im folgenden Schritt 4 entnommenen Produktlösung erforderlich.

Schritt 4: Ventil **21** wird geschlossen, Ventil **22** wird geöffnet und der Kolben **23** fährt in den Zylinder **24** ein. In Schritt 4 wird Produktlösung durch Ventil **22** in Richtung Produktbehälter **16** gefördert. Anschließend wird Ventil **22** geschlossen. Die beschriebenen Schritte werden nun periodisch wiederholt.

In einer solchen Anordnung lässt sich zum Beispiel bei einem Verhältnis von Hubvolumen zu Innenraumvolumen von drei und bei zweifacher Durchführung der Schritte 2 und 3 eine Entnahmekonzentration des Produktstroms von über 80% der Konzentration im Reaktor **1** erreichen.

Der mittelfristige Flüssigkeitsstand im Reaktor **1** lässt sich durch Wahl des Verhältnisses der in Schritt 1 und 4 geförderten Flüssigkeitsvolumina steuern. Dies kann durch entsprechende füllstandsgeregelte Steuerung der Taktzeiten oder der Fördervolumina pro Takt ohne manuelle Eingriffe realisiert werden.

### Variante 2

Im Folgenden wird eine Variante mit zwei Förderorganen und je einem Ein- und Ausgang des Innenraums beschrieben. Sie umfasst mindestens einen Vorratsbehälter, ein Membransystem und einen Reaktor, sowie zwei volumetrisch fördernde Aggregate, zum Beispiel Kolbenpumpen, Schlauch- oder Zahnradpumpen und zwei Ventile, die ebenso wie die Pumpen an eine Taktsteuerung angeschlossen sind. Bei Verwendung von Pumpen mit Absperrfunktion im Stillstand, also zum Beispiel Schlauchpumpen, kann auf die Ventile verzichtet werden.

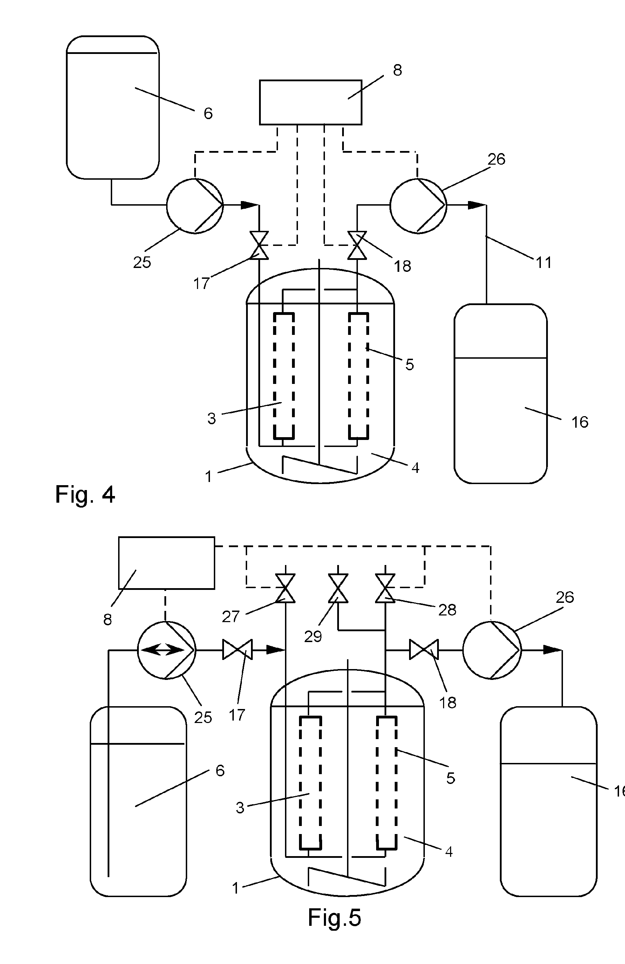

**Fig. 4** zeigt schematisch eine zur Durchführung geeignete Anordnung.

Die Pumpen **25** und **26** fördern abwechselnd und der der jeweils fördernden Pumpe zugewandte Ein- beziehungsweise Ausgang des Innenraums **3** ist offen, während der andere durch eines der Ventile **17**, **18** abgesperrt ist. Innerhalb der zwei Takte umfassenden Periode erfolgt, gesteuert durch die Steuereinheit **8**, ein Drucktakt, in dem Flüssigkeit vom Vorratsbehälter **6** in den Innenraum **3** und von dort über die Membran **5** in den Außenraum **4** gedrückt wird und ein Saugtakt, in dem Flüssigkeit vom Außenraum **4** in den Innenraum **3** gesaugt und von dort zur Produktentnahme gefördert wird.

Je größer das Verhältnis des pro Saugtakt entnommenen Flüssigkeitsvolumens zum Volumen des Innenraums **3** desto höher ist die Produktkonzentration der entnommenen Produktlösung **11**.

Das Verhältnis der pro Periode zugeführten und entnommenen Volumina bestimmt den Verlauf des Flüssigkeitsstandes im Reaktor **1** und kann als Stellgröße einer Standregelung verwendet werden. Bei kontinuierlichen Fermentation sollte dies ein nach kontinuierlicher oder taktweiser Ausschleusung des Biomassenzuwachses zeitlich konstanter Stand sein, bei Fed-Batch-Betrieb wird eine kontrollierte Zunahme des Stands angestrebt.

Durch Variation der Pumpleistungen der beiden Pumpen **25**, **26** können die Dauern der einzelnen Takte unter Einhaltung des gewünschten Verhältnisses der geförderten Volumina variiert werden. So ist es möglich, den Drucktakt mit höherer Pumpleistung und in kürzerer Zeit durchzuführen als den Saugtakt, wodurch sich die Gefahr des Foulings weiter verringert und die gesamte Periodendauer sinkt.

### Variante 3

In dieser in **Fig. 5** schematisch dargestellten, bevorzugten Variante wird eine besonders hohe Produktkonzentration bei niedriger Membranfläche erreicht. Hierzu wird zum Beispiel eine Anordnung mit zwei volumetrisch fördernden Förderorganen **25** und **26** von denen zumindest das erste eine umkehrbare Förderrichtung besitzt und vier getaktete Ventilen **17**, **18**, **27**, **28**, sowie einem Entlüftungsventil **29** verwendet. Zusätzlich zu den Funktionen der Variante 2 wird das Volumen des Innenraums **3** jeweils zwischen den Druck- und Saugtakten entleert. Die Entleerung zwischen Druck- und Saugtakt vermeidet die Verdünnung der im Saugtakt abgezogen Flüssigkeit durch Reste der vom letzten Drucktakt im Innenraum **3** verbliebenen Lösung, die Entleerung zwischen Saug- und Drucktakt erhöht die Ausbeute an Produktlösung **11** pro Periode. Nach Öffnen je eines Belüftungsventils **27**, **28** können die Entleerungen durch die Schwerkraft, durch von einem Pneumatikzylinder geförderte oder aus einem Druckluftnetz entnommene Luft oder, wie in **Fig. 5** gezeigt, mittels der auch in den Saug- und Drucktakten benutzten Pumpen **25**, **26** erfolgen. Die bei der ersteren Entleerung entnommene Flüssigkeit kann in den Vorratsbehälter **6** zurückgefördert werden, während die zweite Entleerung zur Produktentnahme hin erfolgt. Die Betriebsweise enthält insgesamt vier Takte pro Periode, in denen folgende Funktionen ablaufen:

Takt 1: Im Drucktakt wird von Pumpe **25** Flüssigkeit bei geöffnetem Ventil **17** aus dem Vorratsbehälter **6** in den Innenraum **3** gefördert. Zunächst entweicht hierbei die im Innenraum **3** befindliche Luft durch das Entlüftungsventil **29** bis der Innenraum **3** mit Flüssigkeit gefüllt ist. Dann schließt das Entlüftungsventil **29** selbsttätig und es wird Flüssigkeit vom Innenraum in den Außenraum gedrückt.

Takt 2: Im ersten Entleerungstakt wird der Innenraum **3** in den Vorratsbehälter **6** entleert. Dies erfolgt durch Umkehren der Förderrichtung der Pumpe **25** und Öffnen des Ventils **28**, wodurch Flüssigkeit aus dem Innenraum **3** in den Vorratsbehälter **6** strömt und Luft in den Innenraum **3** nachströmt.

Takt 3: Im Saugtakt wird nach Abschalten der Pumpe **25**, Schließung der Ventile **17**, **28** und Öffnung des Ventils **18** zunächst die im Innenraum **3** befindliche Luft und dann durch die Membran **5** angesaugte produktreiche Lösung von Pumpe **26** zur Produktentnahme gefördert.

Takt 4: Im zweiten Entleerungstakt wird nach Öffnen des Ventils **27** die im Innenraum **3** befindliche Produktlösung von der Pumpe **26** zur Produktentnahme gefördert, wobei Luft in den Innenraum **3** nachströmt. Dann werden die Ventile **18** und **27** geschlossen und die Pumpe **26** abgeschaltet. Nach Abfolge der Takte 1-4 ist eine Periode abgeschlossen und der Betrieb wird wieder mit Takt 1 fortgesetzt.

Das Verhältnis der netto pro Periode aus dem Vorratsbehälter **6** und zur Produktentnahme geförderten Flüssigkeitsvolumina bestimmt den längerfristigen Verlauf des Flüssigkeitsstandes im Reaktor **1**. Da während der Entleerungstakte noch kleinere Mengen an Produktlösung in den Innenraum **3** strömen, empfiehlt sich zur Einstellung des angestrebten Verlauf des Flüssigkeitsstandes eine Regelung der Taktdauern anhand einer Standmessung.

Auch in dieser Variante steigt die Produktkonzentration im entnommenen Strom mit dem Verhältnis aus pro Periode entnommener Flüssigkeit zum Volumen des Innenraums **3**. Da gleichzeitig die Wirkung der Rückspülung der Membran **5** mit der Periodendauer abnimmt, ergibt sich für die Periodendauer ein Optimierungsproblem, das im tatsächlichen Betrieb gelöst werden kann.

### Variante 4:

In dieser Variante wird als zugeführte Flüssigkeit eine Suspension von funktionalisierten Partikeln verwendet, bei denen es sich um Partikel mit Ionenaustauschfunktion, Affinitätsfunktionalität, Adsorptionsfuntionalität oder einer Bindefunktionalität für bestimmte Stoffgruppen oder Einzelstoffe handeln kann. Es ist auch eine Vielzahl von Grundmaterialien für diese Partikel bekannt, also zum Beispiel makro- oder mikroporöse Harze, Hydrogele oder anorganische Materialien wie Aktivkohle oder poröse Keramik. Die Partikelgröße wird einerseits so klein gewählt, dass möglichst kurze Beladungszeiten der Partikel vorliegen, andererseits so groß, dass eine Rückhaltung durch die verwendete Membran sichergestellt ist. Die Verwendung von funktionalisierten Partikeln erhöht die Aufnahmekapazität und verringert so die durch das Membranmodul zu transportierenden Mengen an Flüssigkeit. Um eine möglichst große Produktmenge an den funktionalisierten Partikeln zu binden, wird hier die zwischen Innen- und Außenraum ausgetauschte Flüssigkeitsmenge größer gewählt als die durchgesetzte Menge an Suspension. Dies kann auch durch die Abfolge mehrerer Austauschtakte erfolgen, wie in Variante 1 beschrieben. Die funktionalisierten Partikel können nach anschließender externer Desorption und Waschung wieder zur Adsorption verwendet werden.

**Fig. 8** zeigt exemplarisch ein hierzu geeignetes System, das aus einem belüfteten Reaktor **1** mit einem Reaktionsbehälter und einem darin angeordnetem Membransystem **2**, einem Vorratsbehälter für Adsorbenssuspension **6**, einem getakteten Pulsations- und Fördersystem **7**, sowie mindestens zwei als Anschwemmfilter und als Desorber abwechselnd betriebenen Membranfiltersystemen **30 a und 30 b** besteht, auf denen die Partikel als Festbett **35** angeschwemmt, mittels einer durch das Bett geleiteten Desorptionslösung **36** regeneriert, gewaschen und dann zum Wiedereinsatz mit frischer Trägerlösung **37** ausgespült werden können. Die Produktlösung **11** wird als Filtrat gewonnen, gegebenenfalls vereinigt mit der Waschfraktion.

Vor allem in großen Anlagen erfordert das vollständige Entleeren des Innenraums besondere Aufmerksamkeit. Es kann vorteilhaft sein, dann beide Entleerungen in Variante 3 von der gleichen Seite vorzunehmen, wodurch eine geeignete Umschaltung der Entleerungsleitung und mindestens ein zusätzliches Ventil erforderlich werden. In diesem Fall bietet sich auch eine Entleerung durch die Einleitung von Druckluft an.

### Generelle Bemessungskriterien

Die bei der Auslegung einer erfindungsgemäßen Ausführung des Verfahrens festzulegenden Parameter umfassen unter anderem:

### Die Membranfläche

Die Größe der benötigten Membranfläche hängt ab von:
- dem zuzuführenden Flüssigkeitsstrom, der von der benötigten Nährstoffmenge abhängt, sowie dem zu entnehmenden Flüssigkeitsstrom, der wiederum von der Erzeugungsrate und maximal zulässigen Konzentration des Produktes oder inhibierenden Nebenproduktes und deren tatsächlicher Konzentration im entnommenen Produktstrom abhängt. Die Membrandimensionierung richtet sich primär nach der Entnahme, da der Fluss durch die Membran vom Außenin den Innenraum meist geringer ist als der Fluss in umgekehrter Richtung.
- der Filtrierbarkeit der im Außenraum befindlichen Suspension oder Lösung,
- den Eigenschaften der Membran, insbesondere ihrer Permeabilität,
- den Betriebsparametern, insbesondere der Periodendauer und der Durchmischungsintensität im Außenraum.

Eine zuverlässige Dimensionierung der Membranfläche erfordert Pilotversuche mit einer zum Scale Up geeigneten Anordnung und dem realen Stoffsystem. Der Membranflächenbedarf bei Verwendung von zur Rückhaltung von Mikroorganismen geeigneten Mikro- oder Ultrafiltrationsmembran aus handelsüblichem Polymermaterial lässt sich für die bevorzugte Variante 3 folgendermaßen grob abschätzen: Während der Saugtakte wurden in Laborversuchen, die unten beschrieben werden, Austausch-Flüsse von mindestens 60 Liter pro Quadratmeter Membranfläche und Stunde erreicht. Dieser Wert liegt wesentlich höher als Werte für konventionell betriebene Membranbioreaktoren. Geht man von einem Zeitbedarf der Saugtakte in Höhe der Hälfte der Gesamtzeit aus, dann ergibt sich bezogen auf den entnommenen Strom ein mittlerer Wert von 30 Liter pro Quadratmeter Membranfläche und Stunde. Ein Reaktor, der 1 kg Produkt pro Stunde erzeugt, benötigt bei einer Maximalkonzentration des Produkts von 12 g/L im Reaktor und einer Konzentration von 10 g/L im entnommenen Strom demnach etwa 3,3 Quadratmeter Membranfläche.

Sehr viel niedrigere Membranflächen werden bei Verwendung von Mikrosieben als Membran benötigt. Hier lässt sich jedoch kein Richtwert angeben. In Filtrationsanordnungen werden mit hochfrequent rückgespülten Mikrosieben Flüsse von mehreren Tausend Liter pro Quadratmeter Membranfläche und Stunde erreicht.

### Periodendauer und Taktzeiten

Die Periodendauer, die in weiten Grenzen verändert werden kann, richtet sich vor allem nach dem Volumen des Innenraums des Membransystems und der zu entnehmenden Flüssigkeitsmenge. Diese wiederum hängt von der angestrebten Produktkonzentration im entnommenen Strom ab. Besonders kurze Periodendauern werden in Variante 3, das heißt bei Entleerung des Innenraums zwischen den Druck- und Saugtakten erreicht. Geht man von einem Saugtakt pro Periode aus, dann sollte hier die Periodendauer das Zwei- bis Fünffache der Zeit betragen, die sich aus dem Quotienten des Innenraumvolumens und des mittleren entnommenen Flüssigkeitsstroms ergibt. Bei einem Innenraumvolumen von 0,05 Liter und einem entnommenem Produktstrom von 0,3 Liter pro Minute ergibt sich zum Beispiel eine Periodendauer von 20 bis 50 Sekunden. Typische Periodendauern liegen für Polymermembranen im Bereich von 10 Sekunden bis zu einigen Minuten. Bei Verwendung von Mikrosieben liegen die Periodendauern deutlich niedriger, also eher unter zwanzig Sekunden. Die angegebenen Periodendauern gehen davon aus, dass man einerseits relativ hohe Produktkonzentrationen im Entnahmestrom anstrebt und andererseits das Innenraumvolumen auf das technisch mögliche Maß beschränkt wird. Bei geringen Anforderungen an die Produktkonzentration kann die Periodendauer verringert werden, bei ungünstigem Design des Membransystems sind höhere Periodendauern erforderlich. Höhere Periodendauern führen zu erhöhtem Membranfouling.

### Anwendungsbereich

Das Verfahren ist für alle Fermentationsverfahren und enzymatischen Umsetzungen einsetzbar, bei denen das Produkt in der Fermentationsbrühe oder im Enymreaktor in gelöster Form vorliegt. Es eignet sich für Batch- Fed-Batch- und Kontibetrieb und bietet sowohl im Labor- als auch im Pilotmaßstab und in technischen Anlagen große Vorteile. Besonders interessant ist der Einsatz in produktlimitierten Prozessen. Hierzu gehören z.B. die Herstellung von Milchsäure (ab 6 % stark inhibierend), Butanol (ab 4 % stark inhibierend), Itaconsäure, Aminosäuren oder Ethanol. Auch für kontinuierlich betriebene Fermentationsprozesse allgemein, wie z.B. Ethanol, Aceton, Butanol oder Milchsäure [11] bietet das Verfahren wesentliche Vorteile.

### Anwendungsbeispiel: Kontinuierliche Fermentation

In einem kontinuierlich betriebenen Fermenter mit 24 Liter Volumen für die Herstellung von Itaconsäure durch einen Wildstamm des Pilzes Ustilago Maydis mit einer Produktionsrate von 4,8 g/h bei einer maximalen Produktkonzentration von 12 g/L und einer Konzentration von 10 g/L im entnommenen Produktstrom sollte zur Einstellung einer konstanten Konzentration im Fermenter ein Strom von im Mittel 0,48 L/h ausgetauscht und durchgesetzt werden. Es wurde eine einzelne UF-Kapillare von 2 m Länge, 2,6 mm Außen- und 1,3 mm Innendurchmesserdurchmesser (0,016m² Filterfläche) spiralförmig im Außenbereich des Fermenters ähnlich der in **Fig. 2** gezeigten Weise so angeordnet, dass die Anschlüsse über den Deckel erfolgten und der Rührerbetrieb nicht gestört wurde. Der Flüssigkeitszulauf enthielt das Glukose-basierte alkalische Nährmedium. Es wurde wie in **Fig. 4** gezeigt Zwangsförderung durch zwei getaktete Schlauchpumpen gewählt. Je Periode lieferte das System 32 ml Produktlösung, d.h. deutlich mehr als das Gesamt-Flüssigkeitsvolumen in der Kapillare von etwa 8 ml. Der gesamte Takt, d.h. die Summe der Fördertakte der beiden Pumpen dauerte 240 sec. In einer Betriebszeit von 144 h wurden 72 Liter Produktlösung mit einer durchschnittlichen Produktkonzentration von 9,7 g/L gewonnen. Die Konzentration des Produktes lag im Mittel bei über 80% der Konzentration im Fermenter. Die Permeabilität der Membran lag am Ende des Versuchs um weniger als 20% unter dem Anfangswert. Die mit der zugeführten Flüssigkeit aus dem System ausgetragene Nährlösung betrug weniger als 20% der zugeführten Menge. In einer industriellen Fermentation könnte sie nach Abtrennung des Produktes ganz oder teilweise zurückgeführt werden.

### Bezugszeichenliste:

- (1): Reaktor
- (2): Membransystem
- (3): Innenraum des Membransystems (2)
- (4): Außenraum im Reaktorbehälter
- (5): Membran
- (6): Vorratsbehälter
- (7): Pulsations- und Förderungssystem
- (8): Steuereinheit
- (9): Flüssigkeitführende Verbindung für die Flüssigkeitszufuhr bzw.-abfuhr
- (10): Aufarbeitungssystem
- (11): Flüssigkeitsleitung für Produktlösung (Produktstrom)
- (12): Leitung für produktreichen Strom
- (13): Leitung für produktarmen Strom
- (14): Höhendifferenz zwischen dem Flüssigkeitsspiegel im Reaktorbehälter (Außenraum (4))
- (15): Höhendifferenz zwischen dem Flüssigkeitsspiegel im Reaktorbehälter (Außenraum (4)) und dem Flüssigkeitsspiegel im Produktbehälter (16)
- (16): Produktbehälter
- (17): Ventil
- (18): Ventil
- (19): Flüssigkeitszufuhr- und Abfuhrleitung für den Innenraum (3) des Membransystems (2)
- (20): Ventil
- (21): Ventil
- (22): Ventil
- (23): Kolben
- (24): Zylinder
- (25): Pumpen
- (26): Pumpen
- (27): Ventil
- (28): Ventil
- (29): Ventil
- (30 a: und 30 b) Membranfiltersystem

- (31): Plattenmodul
- (32): Rückwand
- (33): Mikrosieb
- (34): Rührer
- (35): Festbett
- (36): Desorptionslösung
- (37): Trägerlösung

## Patentansprüche

1. Verfahren zur Durchführung von Reaktionen mit biologischem Aktivmaterial in einem Reaktor (1) umfassend die Schritte:
a) Bereitstellen eines Reaktors (1) umfassend
i) mindestens einen Behälter mit einem Außenraum (4), in dem sich ein flüssiges Reaktionsmedium, das Produkte erzeugt, befindet, wobei das Reaktionsmedium mindestens ein biologisches Aktivmaterial, ausgewählt aus der Gruppe bestehend aus Enzymen, Zellen, Mikroorganismen und Mischungen hiervon, enthält und
ii) mindestens einem Membransystem (2), das im Innern des Behälters angeordnet ist und das mindestens eine Membran (5) und mindestens einen Innenraum (3) aufweist, wobei mindestens eine Membran (5) in direktem Kontakt mit dem im Behälter befindlichen Reaktionsmedium steht und den Innenraum (3) des Membransystems (2) von dem Außenraum (4) abtrennt und wobei die Membran (5) für das im Reaktionsmedium gelöste Produkt im Wesentlichen permeabel und für das biologische Aktivmaterial im Wesentlichen nicht permeabel ist und
iii) das Membransystem (2), insbesondere der Innenraum (3) des Membransystems (2), mit mindestens einer flüssigkeitführenden Verbindung (9) mit mindestens einem Pulsations- und Förderungssystem (7) verbunden ist
b) Austausch von in dem Innenraum (3) des Membransystems (2) befindlicher Flüssigkeit über die Membran (5) mit im Außenraum (4) befindlicher Produktlösung dadurch, dass der Druck im gesamten Innenraum (3) des Membransystems (2) abwechselnd über den Druck im Außenraum (4) angehoben und unter den Druck im Außenraum (4) abgesenkt wird und
c) Ausschleusung der mit Produkt angereicherten Flüssigkeit aus dem Innenraum (3) des Membransystems (2) und
d) Ergänzen der aus dem Innenraum entnommenen Flüssigkeit durch produktfreie oder produktarme Flüssigkeit, zum Beispiel durch eine Substrat-oder Nährlösung oder einen produktarmen Strom aus der Aufarbeitung der entnommenen Produktlösung.

2. Verfahren gemäß Anspruch 1, **gekennzeichnet dadurch, dass** das im zeitlichen Mittel aus dem Außenraum (4) in den Innenraum (3) durch die Membran (5) tretende Flüssigkeitsvolumen höchstens ebenso groß ist wie das im zeitlichen Mittel aus dem Innenraum (3) in den Außenraum (4) durch die Membran (5) tretende Flüssigkeitsvolumen.

3. Verfahren gemäß Anspruch 1 oder 2 **gekennzeichnet dadurch, dass** die über den Innenraum (3) zugeführte Flüssigkeit mindestens ein zur Erzeugung der Produkte in dem Reaktionsmedium benötigtes Edukt oder Substrat enthält oder, dass die über den Innenraum (3) zugeführte Flüssigkeit zumindest teilweise aus einer Aufarbeitung der in Schritt c) ausgeschleusten Flüssigkeit stammt und bezüglich der Produktkonzentration gegenüber der ausgeschleusten, produktangereicherten Flüssigkeit abgereichert ist.

4. Verfahren gemäß mindestens einem der vorangehenden Ansprüche, **gekennzeichnet dadurch, dass** der Austausch in Schritt b) durch eine periodisch die Richtung wechselnde Durchströmung der Membran (5) und des Innenraums (3) durch die Aufprägung eines periodisch wechselnden Drucks im Innenraum (3) erreicht wird, wobei der Druck im gesamten Innenraum (3) abwechselnd über den Druck im Außenraum (4) angehoben und unter diesen abgesenkt wird und die Druckniveaus und Taktzeiten und die Fläche der Membran(5) entsprechend der Membranpermeabilität so gewählt werden, dass einerseits ein zur Ausschleusung des Produktes ausreichender Flüssigkeitsaustausch über die Membran (5) stattfindet, andererseits im längerfristigen Mittel der gewünschte Verlauf des Füllstandes im Reaktor erreicht wird.

5. Verfahren gemäß mindestens einem der vorangehenden Ansprüche, **gekennzeichnet dadurch, dass** der Austausch in Schritt b) durch eine periodisch die Richtung wechselnde Durchströmung der Membran (5) durch abwechselnde, taktweise Zwangsförderung von Flüssigkeit in den Innenraum (3) des Membransystems (2) hinein und von mit Produkt angereicherter Flüssigkeit aus dem Innenraum (3) heraus erfolgt, wobei der Druck im gesamten Innenraum (3) abwechselnd über den Druck im Außenraum (4) angehoben und unter diesen abgesenkt wird und die Taktvolumina und Taktzeiten und die Membranfläche entsprechend der Membranpermeabilität so gewählt werden, dass einerseits ein zur Ausschleusung des Produktes ausreichender Flüssigkeitsaustausch über die Membran (5) stattfindet, andererseits im längerfristigen Mittel der gewünschte Verlauf des Füllstandes im Reaktor erreicht wird.

6. Verfahren gemäß mindestens einem der vorangehenden Ansprüche, **gekennzeichnet dadurch, dass** der Innenraum (3) des Membransystems (2) periodisch belüftet und von Flüssigkeit entleert wird und die entnommene Flüssigkeit entsprechend ihrer Zusammensetzung dem Innenraum (3) später wieder zugeführt oder mit der entnommenen Produktlösung ausgeschleust wird.

7. Verfahren gemäß mindestens einem der vorangehenden Ansprüche, **gekennzeichnet dadurch, dass** die Taktung der zur Förderung benutzten Ventile und Pumpen so erfolgt, dass nicht in jedem Takt Produktlösung entnommen wird, sondern in einem Teil der Takte nur Flüssigkeit zwischen Innenraum (3) und Außenraum (4) ausgetauscht wird.

8. Verfahren gemäß mindestens einem der vorangehenden Ansprüche, **gekennzeichnet dadurch, dass** als Membran (5) ein oder mehrere sogenannte Mikrosiebe verwendet werden, vorzugsweise mit einem maximalen Porendurchmesser oder einer maximalen Schlitzweite von 10 Mikrometern.

9. Verfahren gemäß mindestens einem der vorangehenden Ansprüche, **gekennzeichnet dadurch, dass** sich an die Ausschleusung der mit Produkt angereicherten Flüssigkeit aus dem Innenraum (3) des Membransystems (2) in Schritt c) eine Produktaufarbeitung anschließt, vorzugsweise durch Verwendung ein oder mehrerer weiterer Membranverfahren, wobei weiter bevorzugt die an Produkt abgereicherte Flüssigkeit ganz oder teilweise über den Innenraum (3) des Membransystems (2) in Schritt b) dem Außenraum (4) des Reaktorbehälters zugeführt wird.

10. Verfahren gemäß mindestens einem der vorangehenden Ansprüche, **gekennzeichnet dadurch, dass** als Produkt ein Alkohol, vorzugsweise ein aliphatischer Alkohol, insbesondere Ethanol oder Butanol erzeugt wird, oder, dass als Produkt eine organische Säure, vorzugsweise Itaconsäure erzeugt wird, oder, dass als Produkt ein monoklonaler Antikörper erzeugt wird.

11. Verfahren gemäß mindestens einem der vorangehenden Ansprüche, **gekennzeichnet dadurch, dass** die in Schritt b) im Innenraum (3) des Membransystems (2) befindliche Flüssigkeit eine Suspension von Adsorbenspartikeln ist, wobei die für die Membran (5) für die Adsorbenspartikel im Wesentlichen nicht permeabel sind und wobei die Adsorbenspartikel das Produkt und/oder ein inhibierendes Nebenprodukt binden und vorzugsweise die beladenen Partikel nach der Beladung ausgeschleust und ganz oder teilweise regeneriert und dann wieder eingesetzt werden.

12. Bioreaktor umfassend
a) mindestens einen Behälter mit einem Außenraum (4) zur Aufnahme eines flüssigen Reaktionsmediums,
b) mindestens ein Membransystem (2), das im Innern des Behälters angeordnet ist und das mindestens eine Membran (5) und mindestens einen Innenraum (3) aufweist, wobei mindestens eine Membran (5) den Innenraum (3) des Membransystems (2) von dem Außenraum (4) abtrennt und wobei das Membransystem (2) mit mindestens einem Pulsations- und Fördersystem (7) verbunden ist.

13. Bioreaktor gemäß Anspruch 12, **dadurch gekennzeichnet, dass** er zusätzlich ein Produktaufarbeitungssystem (10) aufweist.

14. Bioreaktor gemäß Anspruch 12 oder 13, **dadurch gekennzeichnet, dass** das Pulsations- und Fördersystem (7) über mindestens eine gegenüber dem Außenraum (4) flüssigkeitsdichte flüssigkeitführende Verbindung (9) mit dem Innenraum (3) des Membransystems (2) verbunden ist, vorzugsweise ist das Pulsations- und Fördersystem (7) über mindestens eine Flüssigkeitszuleitung und eine Flüssigkeitsableitung mit dem Innenraum (3) des Membransystems (2) verbunden.

15. Verwendung eines Bioreaktors gemäß mindestens einem der vorangehenden Ansprüche 12 bis 14 für biochemische Reaktionen, insbesondere Fermentationen, enzymatische Umsetzungen oder die Produktion monoklonaler Antikörper.
